(19) Europäisches Patentamt
European Patent Office
Office européen des brevets

(11) Numéro de publication : **0 512 899 A1**

# (12) DEMANDE DE BREVET EUROPEEN

(21) Numéro de dépôt : **92401225.5**

(22) Date de dépôt : **30.04.92**

(51) Int. Cl.[5] : **C07D 311/72,** C07D 405/12, C07D 407/12, C07D 409/12, C07D 413/12, C07D 417/12, C07D 471/04, C07D 419/12, A61K 31/355

(30) Priorité : **03.05.91 FR 9105418**

(43) Date de publication de la demande :
**11.11.92 Bulletin 92/46**

(84) Etats contractants désignés :
**AT BE CH DE DK ES FR GB GR IT LI LU NL PT SE**

(71) Demandeur : **ADIR ET COMPAGNIE**
**1 rue Carle Hébert**
**F-92415 Courbevoie Cédex (FR)**

(72) Inventeur : **Le Baut, Guillaume**
**5 rue de la Baugerie**
**F-44230 Saint Sebastien sur Loire (FR)**
Inventeur : **Babingui, Jean-Paul**
**10 rue de Briord**
**F-44000 Nantes (FR)**
Inventeur : **Courant, Jacqueline**
**31 avenue de l'Ombrie**
**F-44000 Nantes (FR)**
Inventeur : **Robert, Jean-Michel**
**145-147 rue Bonne Garde**
**F-44000 Nantes (FR)**
Inventeur : **Renard, Pierre**
**50 avenue de Villeneuve l'Etang**
**F-78000 Versailles (FR)**
Inventeur : **Caignard, Daniel-Henri**
**69 bis rue Brancion**
**F-75015 Paris (FR)**
Inventeur : **Renaud de la Faverie, Jean-François**
**7 rue des Erables - Rocquencourt**
**F-78150 Le Chesnay (FR)**
Inventeur : **Adam, Gérard**
**9 Clos du Mesnil, Route du Pecq**
**F-78600 Le Mesnil le Roi (FR)**

## (54) Nouveaux composés benzopyraniques, leur procédé de préparation et les compositions pharmaceutiques qui les contiennent.

(57) Composés de formule I :

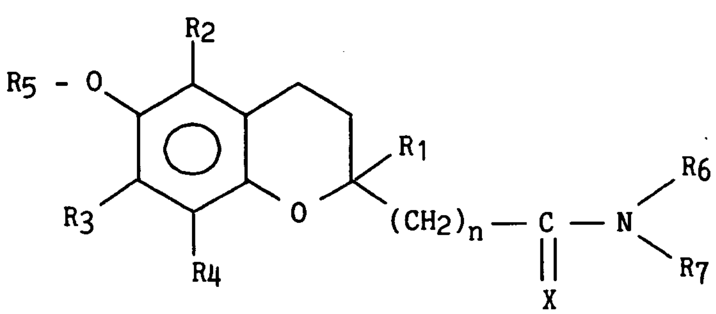

(I)

dans laquelle $R_1$, $R_2$, $R_3$, $R_4$, $R_5$, $R_6$, $R_7$, X, et n sont définis dans la description,
leurs isomères optiques,
et leurs sels d'addition à une base ou à un acide pharmaceutiquement acceptable.
Médicaments.

EP 0 512 899 A1

La présente invention concerne de nouveaux composés benzopyraniques, leur procédé de préparation et les compositions pharmaceutiques qui les contiennent.

On connait de nombreux composés benzopyraniques, notamment les tocophérols, composés vitaminiques E dotés en particulier de propriétés antioxydantes. De nombreux composés du tocophérol ont été préparés, résultant d'une modification de la chaine latérale de nature alcanoïque, sans augmentation de l'activité antioxydante. D'autres composés benzopyraniques, comme l'acide (6-hydroxy-2,5,7,8-tétraméthylchroman-2-yl) carboxylique possèdent une bonne activité antioxydante, mais uniquement à visée industrielle, n'ayant pas reçu d'applications thérapeutiques.

Plus récemment, la demande de brevet WO 88/08424 a décrit d'autres composés de l'acide chromane-2-carboxylique et plus généralement d'acides (chroman-2-yl) alkylcarboxyliques, présentant des propriétés antioxydantes intéressantes.

La demanderesse a maintenant découvert de nouveaux composés benzopyraniques, et plus particulièrement de l'acide (benzopyran-2-yl) carboxylique ou acide (chroman-2-yl) carboxylique possédant une activité anti-oxydante nettement supérieure à celles des composés de la demande WO 88/08424 qui constituent l'art antérieur le plus proche.

Plus spécifiquement l'invention concerne de nouveaux composés benzopyraniques répondant à la formule générale (I) :

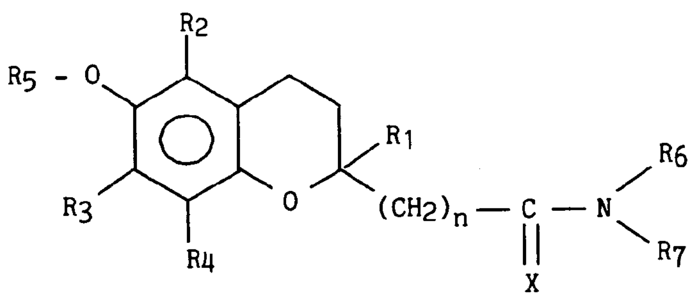

(I)

dans laquelle :

n représente un nombre entier égal à 0 ou 1,

X représente un atome d'oxygène ou 2 atomes d'hydrogène,

$R_1$, $R_2$, $R_3$, $R_4$, identiques ou différents, représentent chacun indépendamment l'un de l'autre un atome d'hydrogène ou un radical alkyl inférieur $R_a$-, où Ra représente un groupement alkyle linéaire ou ramifié de 1 à 8 atomes de carbone,

$R_5$ représente :

- un atome d'hydrogène,
- un groupement alkyle inférieur $R_a$-,
- un groupement acyle inférieur $R_a$-CO-,
- un groupement alcoxyalkyle de forme $R_a$-O-$R_b$-,
- un groupement alcoxycarbonyle de forme $R_a$-O-CO-,
- un groupement alcoxycarbonylalkyle de forme $R_a$-O-CO-$R_b$-,
- un groupement carboxylalkyle de forme HOOC-$R_a$-,

où $R_a$ et $R_b$, identiques ou différents, représentent chacun indépendamment l'un de l'autre, un radical alkyle linéaire ou ramifié comportant de 1 à 8 atomes de carbone,

$R_5$ et $R_7$

- soit :

forment ensemble avec l'atome d'azote qui les porte un groupement E ou un groupement E substitué avec E représentant un groupement hétérocyclique spirannique de 8 à 12 chaînons incluant dans son squelette carboné de 1 à 4 hétéroatomes choisis parmi azote, soufre, et oxygène,

- soit :

identiques ou différents, représentent chacun indépendamment l'un de l'autre :

- un atome d'hydrogène,
- un groupement alkyle inférieur Ra- ou un groupement alkyle inférieur Ra- substitué,
- un groupement alcényle inférieur ou un groupement alcényle inférieur substitué, où le groupement alcényle représente un hydrocarbure insaturé, linéaire ou ramifié, comprenant 2 à 8 atomes de carbone,
- un groupement A-$(CH_2)_m$- ou un groupement A-$(CH_2)_m$- substitué, où m est un nombre entier égal à 0,1, ou 2 et A représente un groupement cycloalkyle comportant p atomes de carbone avec p étant un

nombre entier de 3 à 7 étant entendu que si p est 3 ou 4 alors m peut être 0, 1 ou 2 et si p est 5,6, ou 7 alors m ne peut être que 1 ou 2,

- un groupement alcoxyalkyle de forme $R_a$-O-$R_b$- ou un groupement alcoxyalkyle de forme $R_a$-O-$R_b$- substitué, où $R_a$ et $R_b$, identiques ou différents, représentent un radical alkyle inférieur, linéaire ou ramifié, de 1 à 8 atomes de carbone,
- un groupement alcoxycarbonylalkyle de forme $R_a$-O-CO-$R_b$- ou un groupement alcoxycarbonylalkyle de forme $R_a$-O-CO-$R_b$- substitué, avec $R_a$ et $R_b$ tels que définis ci-dessus,
- un groupement B-$(CH_2)_q$- ou un groupement B-$(CH_2)_q$- substitué, où q est un nombre entier égal à 0, 1, 2, ou 3 et B représente un radical naphtalène, 1,3-dioxane , pyrane, ou benzopyrane,
- un groupement E-$(CH_2)_q$- ou un groupement E-$(CH_2)$q- substitué, avec q et E tels que définis précédemment,
- un groupement phényl-$(CH_2)_q$- ou un groupement phényl-$(CH_2)_q$- substitué, avec q tel que défini ci-dessus,
- un groupement hétéroaryl-$(CH_2)_q$- ou un groupement hétéroaryl-$(CH_2)_q$- substitué, avec q tel que défini ci-dessus et où l'hétéroaryle est choisi parmi :

furane, quinoléine, isoquinoléine, pyridine, thiophène, thiazole, isothiazole, oxazole, isoxazole, naphtyridine, benzofurane, β-carboline , ou γ-carboline,

- un des radicaux $D_1$ à $D_9$ suivants ou un des radicaux $D_1$ à $D_9$ suivants substitué :

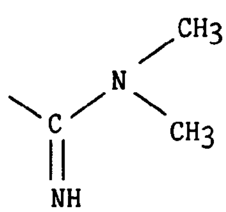

,

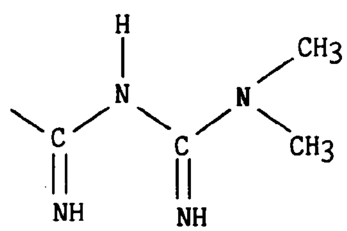

,

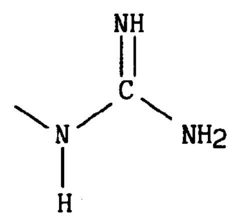

,

D1 D2 D3

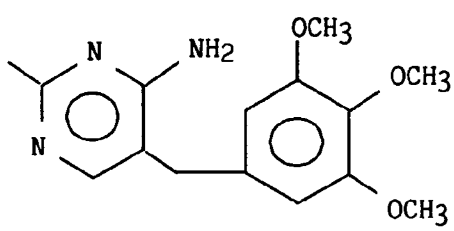

,

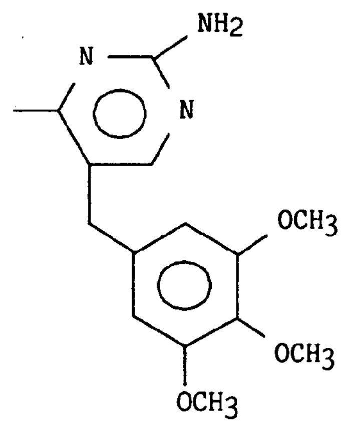

,

D4 D5

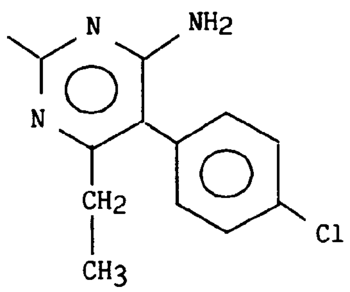

D6

,

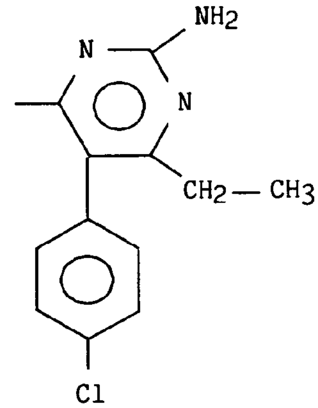

D7

,

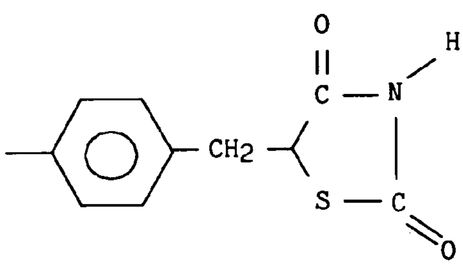

D8

,

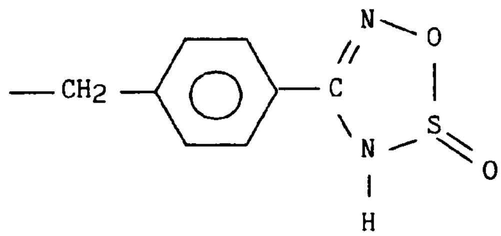

D9

,

étant entendu que

lorsque l'un des substituants $R_6$ ou $R_7$ représente un atome d'hydrogène, un groupement alkyle inférieur comportant au maximum 3 atomes de carbone, un groupement phényl-$(CH_2)_{q'}$- non substitué, un groupement phényl-$(CH_2)_{q'}$- substitué par 1 ou 2 radicaux, un groupement pyridinyl-$(CH_2)_q$- non substitué, ou un groupement pyridinyl-$(CH_2)_q$- substitué par 1 ou 2 radicaux, avec q tel que défini précédemment et q' étant un nombre entier égal à 1, 2, ou 3,

alors l'une au moins des 2 conditions suivantes est vérifiée :

- ou bien l'autre substituant, $R_6$ ou $R_7$ selon le cas, ne représente pas : un atome d'hydrogène, un groupement alkyle inférieur comportant au maximum 3 atomes de carbone, un groupement phényl-$(CH_2)_{q'}$- non substitué, un groupement phényl-$(CH_2)_{q'}$- substitué par 1 ou 2 radicaux, un groupement pyridinyl-$(CH_2)_q$- non substitué, un groupement pyridinyl-$(CH_2)_q$- substitué par 1 ou 2 radicaux, avec q et q' tels que définis précédemment,
- ou bien le substituant $R_5$ ne représente pas un atome d'hydrogène, un groupement alkyle inférieur comportant au maximum 4 atomes de carbone, ou un groupement acyle inférieur $R'_5$-CO- avec $R'_5$ représentant un radical alkyle inférieur comportant au maximum 4 atomes de carbone,

étant entendu que

lors de cette description de la formule générale (I), le terme "substitué" affectant les groupements tels que définis précédemment : alkyle inférieur Ra-, alcényle inférieur, A-$(CH_2)_m$-, alcoxyalkyle $R_a$-O-$R_b$-, alcoxycarbonylalkyle $R_a$-O-CO-$R_b$-, B-$(CH_2)_q$-, E-$(CH_2)_q$-, phényl-$(CH_2)_q$-, phényl-$(CH_2)_{q'}$-, hétéroaryl-$(CH_2)_q$-, $D_1$ à $D_9$, signifie, lorsque cela n'est pas précisé, que ces groupements peuvent être substitués par un ou plusieurs radicaux, identiques ou différents, et représentant chacun indépendamment l'un de l'autre :

un groupement alkyle inférieur $R_c$-,

alcoxy inférieur $R_c$-O-,
acyle inférieur $R_c$-CO-,
trifluorométhyle,
carboxyle,
hydroxyle,
amino,
amino substitué par 1 ou 2 groupement alkyles inférieur $R_c$-,
nitro,
oxo,
phényle,
alkylthio inférieur $R_c$-S-,
thiol,
ou un atome d'halogène,
où $R_c$ représente un groupement alkyle linéaire ou ramifié de 1 à 6 atomes de carbone,
leurs isomères optiques,
ainsi que, le cas échéant, leurs sels d'addition à un acide ou à une base pharmaceutiquement acceptable.

Parmi les acides ou les bases pharmaceutiquement acceptables que l'on peut utiliser pour salifier les composés de l'invention, on peut citer, à titre d'exemples et de façon non limitative, les acides chlorydrique, bromhydrique, sulfurique, nitrique, oxalique, malique, maléique, succinique, tartrique, méthanesulfonique, camphorique, camphosulfonique, la soude, la potasse, la triéthylamine, la diéthylamine, l'éthanolamine, l'arginine, la lysine, et la diéthanolamine.

L'invention s'étend également au procédé d'obtention des composés de formule (I), caractérisé en ce que l'on utilise comme matière première un composé de formule (II) :

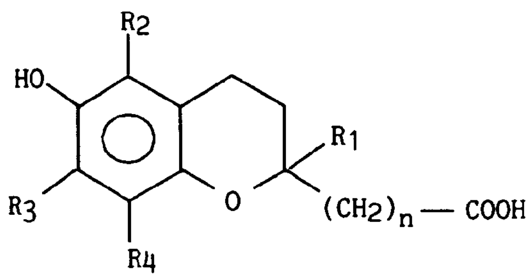

(II)

avec $R_1$, $R_2$, $R_3$, $R_4$, et n ayant la même définition que dans la formule générale (I),
que l'on peut éthérifier ou estérifier, en milieu basique anhydre, par un composé $R_{5''}$-Hal ou $R_{5''}$-O-$R_{5''}$, où Hal représente un atome d'halogène et où $R_{5''}$ représente un groupement alkyle inférieur $R_a$-, un groupement acyle inférieur $R_a$-CO-, un groupement alcoxyalkyle de forme $R_a$-O-$R_b$-, un groupement alcoxycarbonyle de forme $R_a$-O-CO-, un groupement alcoxycarbonylalkyle de forme $R_a$-O-CO-$R_b$-, un groupement carboxylalkyle de forme HOOC-$R_a$-, formules dans lesquelles $R_a$ et $R_b$, identiques ou différents, représentent chacun indépendamment l'un de l'autre un groupement alkyle inférieur, linéaire ou ramifié, comprenant de 1 à 8 atomes de carbone, pour obtenir un composé de formule (III) :

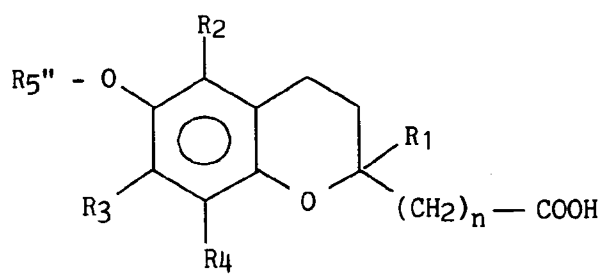

(III)

avec $R_1$, $R_2$, $R_3$, $R_4$, n, et $R_{5''}$ tels que définis précédemment, qui est transformé en son chlorure par action d'un agent d'halogénation, puis traité, dans un solvant approprié, en présence d'un agent alcalin, par une amine de formule (IV) :

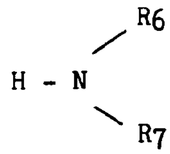

(IV)

avec $R_6$ et $R_7$ ayant la même signification que dans la formule (I) pour obtenir un composé de formule ($I_a$) :

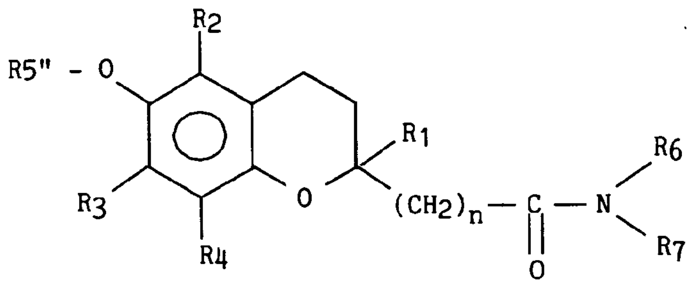

($I_a$)

avec $R_1$, $R_2$, $R_3$, $R_4$, $R_6$, $R_7$, n, et $R_{6''}$ tels que définis précédemment, cas particulier des composés de formule (I) pour lesquels $R_6$ représente un groupement $R_{6''}$ et X représente un atome d'oxygène,

que l'on peut ensuite :

- soit réduire par action d'un hydrure mixte de métal alcalin en un composé de formule ($I_b$) :

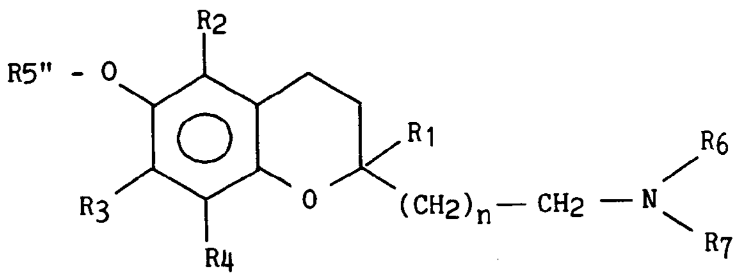

($I_b$)

avec $R_1$, $R_2$, $R_3$, $R_4$, $R_6$, $R_7$, n, et $R_{6''}$ tels que définis précédemment, cas particulier des composés de formule (I) pour lesquels $R_6$ représente un groupement $R_{6''}$ et X représente deux atomes d'hydrogène,

- soit saponifier par action d'un hydroxyde de métal alcalin ou alcalino-terreux en un composé de formule ($I_c$) :

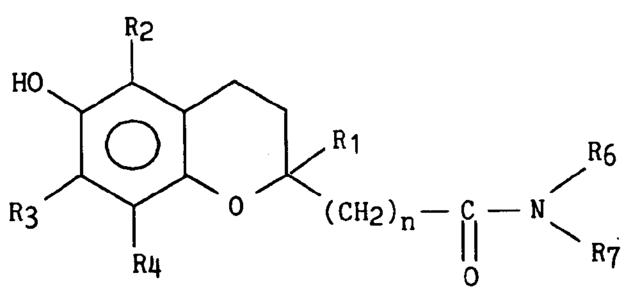

($I_c$)

avec $R_1$, $R_2$, $R_3$, $R_4$, $R_6$, $R_7$, et n tels que définis précédemment,

cas particulier des composés de formule (I) pour lesquels $R_5$ représente un atome d'hydrogène et X représente un atome d'oxygène, composé ($I_c$) qui peut être ensuite :

- ou bien réduit par action d'un hydrure mixte de métal alcalin en un dérivé de formule ($I_d$) :

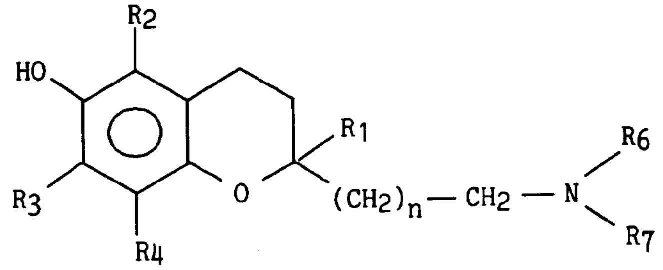

(Id)

avec $R_1$, $R_2$, $R_3$, $R_4$, $R_6$, $R_7$, et n tels que définis précédemment, cas particulier des composés de formule (I) pour lesquels $R_5$ représente un atome d'hydrogène et X représente 2 atomes d'hydrogène,

- ou bien éthérifié ou estérifié, afin d'obtenir, si on le désire, un groupement $R_5$ différent de celui présent dans la formule (Ib), par action d'un dérivé de formule $R_{5'''}$-O-$R_{5'''}$ ou $R_{5'''}$-Hal, où Hal représente un atome d'halogène et $R_{5'''}$ représente un groupement acyle inférieur $R_a$-CO-, un groupement alcoxyalkyle de forme $R_a$-O-$R_b$-, un groupement alcoxycarbonylalkyle de forme $R_a$-O-CO-$R_b$-, ou un groupement carboxylalkyle de forme HOOC-$R_a$-, avec $R_a$ et $R_b$ tels que définis précédemment, pour obtenir un composé de formule ($I_e$) :

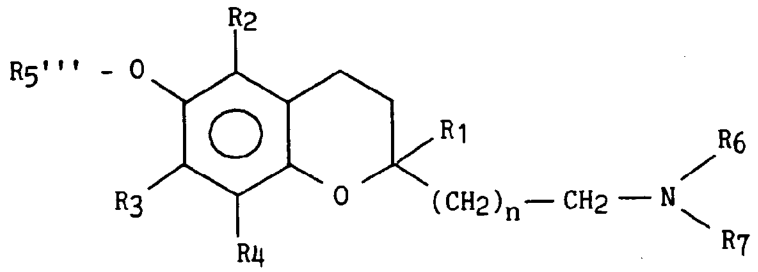

(Ie)

avec $R_1$, $R_2$, $R_3$, $R_4$, $R_5$, $R_7$, n, et $R_{5'''}$ tels que définis précédemment, cas particulier des composés de formule (I) pour lesquels $R_5$ représente un groupement $R_{5'''}$ et X représente 2 atomes d'hydrogène.

Les composés de formule (Ia), (Ib), (Ic), (Id), et (Ie) forment l'ensemble des composés de formule (I) qui peuvent être, si on le désire, purifiés et/ou séparés en leurs isomères optiques.

Ces composés de formule (I) peuvent également être, le cas échéant, transformés en leurs sels d'addition à une base ou à un acide pharmaceutiquement acceptable.

Comparés aux composés de l'art antérieur, ces composés de la présente invention possèdent de façon surprenante des propriétés antioxydantes très importantes. Les études pharmacologiques ont notamment montré que ces composés étaient doués d'activités protectrices remarquables dans le cadre des processus de peroxydations des lipides cellulaires et des lipoprotéines de faible densité (LDL).

Ces activités sont pour certains des composés de l'invention 100 fois supérieures à celle du composé le plus proche de l'art antérieur, c'est à dire l'exemple 102 de la demande WO 88/08424.

Par ailleurs, certains composés de la présente invention présentent la particularité d'avoir un puissant effet inhibiteur de la biosynthèse des prostanoïdes, lesquels proviennent de composés peroxidés, qui sont des générateurs potentiels de radicaux libres, effet inhibiteur que ne possède pas le composé le plus proche de l'art antérieur.

On peut donc attendre des composés de l'invention, qui présentent à la fois des propriétés inhibitrices de la peroxydation lipidique et de la biosynthèse des prostanoïdes, une action particulièrement nouvelle et bénéfique dans les affections où interviennent non seulement une peroxydation des lipides membranaires mais aussi un dérèglement de la synthèse des prostanoïdes.

Les composés de la présente invention peuvent ainsi être utilisés dans le traitement ou la prévention des affections dues ou reliées à de tels phénomènes de peroxydation et de dérèglements de la biosynthèse des prostanoïdes, c'est à dire les désordres ischémiques centraux ou périphériques, les maladies inflammatoires, l'arthrite rhumatoïde, les maladies métaboliques, l'athérome, l'artériosclérose, les maladies respiratoires, l'asthme, l'emphysème, les maladies d'origine immunologique, le lupus érythémateux, les réactions allergiques, certains cancers, le vieillissement cérébral ou cutané ainsi que dans la prévention et le traitement des dommages dus aux traumatismes chirurgicaux tels que la reperfusion d'organes.

La présente invention a également pour objet les compositions pharmaceutiques contenant les composés

de formule I, ou un de leurs sels d'addition à un acide ou une base pharmaceutiquement acceptable, seul ou en combinaison avec un ou plusieurs excipients inertes non toxiques.

Parmi les compositions pharmaceutiques selon l'invention, on pourra citer plus particulièrement, à titre d'exemples et de façon non limitative, celles qui conviennent à l'administration orale, parentérale, nasale, rectale, perlinguale, oculaire ou pulmonaire et notamment les préparations injectables, les aérosls, les gouttes oculaires ou nasales, les comprimés simples, pelliculés ou dragéifiés, les gélules, les capsules, les crèmes, pommades, et gels dermiques.

La posologie utile varie selon l'âge et le poids du patient, la voie d'administration, la nature de l'affection et des traitements éventuellement associés et s'échelonne entre 0,5 mg et 2 grammes par 24 heures.

Les exemples qui suivent illustrent l'invention et ne la limitent en aucune façon.

La matière première est décrite dans la littérature ou est facilement accessible à l'homme de l'art.

Les spectres infra rouge sont réalisés en pastille de bromure de potassium renfermant environ 1 % du produit à analyser.

**Exemple 1 :**

**N-isobutyl N-(4,6-diméthylpyridin-2-yl) (6-acétoxy-3,4-dihydro-2,5,7,8-tétraméthyl-2H[1]-benzopyran-2-yl) carboxamide**

**STADE A :**

**Acide (6-acétoxy-3,4-dihydro-2,5,7,8-tétraméthyl-2H[1]-benzopyran-2-yl)carboxylique**

Dissoudre 50 grammes (0,2 mole) d'acide (6-hydroxy-3,4-dihydro-2,5,7,8-tétraméthyl-2H[1]-benzopyran-2-yl) carboxylique dans 150 $cm^3$ de pyridine anhydre. Ajouter goutte à goutte 9,4 $cm^3$ (0,1 mole) d'anhydride acétique sous courant d'azote. Agiter pendant 2 heures à une température de 30°C. Après refroidissement, verser le mélange sur de la glace, extraire le produit attendu par de l'éther éthylique, laver la phase organique par une solution d'acide chlorhydrique 0,2N , puis à l'eau jusqu'à neutralité. Après évaporation du solvant, on recueille une masse huileuse qui cristallise après trituration dans l'éther diisopropylique.

**STADE B :**

**N-isobutyl N-(4,6-diméthylpyridin-2-yl) (6-acétoxy-3,4-dihydro-2,5,7,8-tétraméthyl-2H[1]-benzopyran-2-yl) carboxamide**

Dissoudre 3,21 grammes (11,21 mmoles) du composé du stade A de l'exemple 1 précédent dans 40 $cm^3$ de benzène anhydre, ajouter 1,2 $cm^3$ de chlorure de thionyle. Chauffer au reflux pendant 3 heures, laisser refroidir. Chasser le solvant sous vide en éliminant l'excès de chlorure de thionyle. Dissoudre le chlorure d'acide ainsi obtenu dans 25 $cm^3$ de dichloroéthane.

Dans un autre récipient, dissoudre 2 grammes (11,21 mmoles) de N-(4,6-diméthylpyridin-2-yl) isobutylamine et 4,7 $cm^3$ de triéthylamine dans 25 $cm^3$ de dichloroéthane, puis verser goutte à goutte sur ce mélange la solution de chlorure d'acide précédente, chauffer au reflux pendant 3 heures puis évaporer le solvant sous vide. Reprendre le résidu obtenu par 30 $cm^3$ d'eau, neutraliser par une solution de bicarbonate de soude, extraire par du dichlorométhane, laver la phase organique puis sécher. Après élimination du solvant, purifier le produit en utilisant les techniques classiques de séparation chromatographique ou cristallographique.

On obtient le produit du titre :

Rendement : 55 %

- Point de fusion : 90 - 92°C
- Caractéristiques spectrales en infra rouge :

| | |
|---|---|
| $\nu$C=O (ester) | : 1750 $cm^{-1}$ |
| $\nu$C=O (amide) | : 1650 $cm^{-1}$ |
| $\nu$C=C, C=N | : 1600, 1550 $cm^{-1}$ |

**Exemple 2 :**

**N-isobutyl N-(4,6-diméthylpyridin-2-yl) (6-hydroxy-3,4-dihydro-2,5,7,8-tétraméthyl-2H[1]-benzopyran-2-yl) carboxamide**

Dissoudre 2,6 grammes (5,74 mmoles) du composé de l'exemple 1 dans 80 $cm^3$ d'éthanol à 60 %, ajouter sous azote 14 $cm^3$ de soude 2,5N. Agiter pendant 3 heures puis diluer le mélange à l'eau et acidifier avec de l'acide acétique. Extraire le produit avec du dichlorométhane, laver la phase organique à l'eau, sécher sur sulfate de sodium anhydre puis évaporer le solvant. Reprendre l'huile obtenue par 15 $cm^3$ d'éther isopropylique et isoler le produit en utilisant les techniques classiques de séparation chromatographique ou cristallographique.

On obtient le produit du titre :

Rendement : 96 %

- Point de fusion : 159 - 160°C
- Caractéristiques spectrales en infra rouge :

νC=O : 1360 $cm^{-1}$
νOH : 3600 - 3200 $cm^{-1}$
νC=C, C=N : 1605, 1560 $cm^{-1}$

**Exemple 3 :**

**N-(2,4,6-triméthylphényl) (6-acétoxy-3,4-dihydro-2,5,7,8-tétraméthyl-2H[1]-benzopyran-2-yl) carboxamide**

En remplaçant dans le stade B de l'exemple 1 la N-(4,6-diméthylpyridin-2-yl) isobutylamine par la 2,4,6-triméthylaniline, on obtient le produit du titre :

Rendement : 73,7 %

- Point de fusion : 148 - 149°C
- Caractéristiques spectrales en infra rouge :

νC=O (ester) : 1750 $cm^{-1}$
νC=O (amide) : 1680 $cm^{-1}$
νNH : 3400 $cm^{-1}$
δNH : 1495 $cm^{-1}$

**Exemple 4 :**

**N-(2,4,6-triméthylphényl) (6-hydroxy-3,4-dihydro-2,5,7,8-tétraméthyl-2H[1]-benzopyran-2-yl) carboxamide**

En opérant comme dans l'exemple 2, mais en remplaçant le composé de l'exemple 1 par le composé de l'exemple 3, on obtient le produit du titre :

Rendement : 95 %

- Point de fusion : 163 - 164°C
- Caractéristiques spectrales en infra rouge :

νC=O : 1670 $cm^{-1}$
νNH : 3400 $cm^{-1}$
νOH : 3440 $cm^{-1}$
δNH : 1500 $cm^{-1}$

**Exemple 5 :**

**N-butyl N-(4,6-diméthylpyridin-2-yl) (6-acétoxy-3,4-dihydro-2,5,7,8-tétraméthyl-2H[1]-benzopyran-2-yl) carboxamide**

En remplaçant dans le stade B de l'exemple 1 la N-(4,6-diméthylpyridin-2-yl) isobutylamine par la N-(4,6-diméthylpyridin-2-yl) butylamine, on obtient le produit du titre :
Rendement : 55,6 %.

**Exemple 6 :**

**N-butyl N-(4,6-diméthylpyridin-2-yl) (6-hydroxy-3,4-dihydro-2,5,7,8-tétraméthyl-2H[1]-benzopyran-2-yl) carboxamide**

En opérant comme dans l'exemple 2, mais en remplaçant le composé de l'exemple 1 par le composé de l'exemple 5, on obtient le produit du titre :

Rendement : 78 %

- Point de fusion : 144 - 145°C
- Caractéristiques spectrales en infra rouge :

νC=O : 1630 $cm^{-1}$
νOH : 3600 - 3300 $cm^{-1}$

νC=C, C=N : 1600, 1560 $cm^{-1}$

**Exemple 7 :**

**N-(3,4,5-triméthoxyphényl) (6-acétoxy-3,4-dihydro-2,5,7,8-tétraméthyl-2H[1]-benzopyran-2-yl) carboxamide**

En remplaçant dans le stade B de l'exemple 1 la N-(4,6-diméthylpyridin-2-yl) isobutylamine par la 3,4,5-triméthoxyaniline, on obtient le produit du titre :

Rendement : 91 %
- Point de fusion : 65 - 68°C

**Exemple 8 :**

**N-(3,4,5-triméthoxyphényl) (6-hydroxy-3,4-dihydro-2,5,7,8-tétraméthyl-2H[1]-benzopyran-2-yl) carboxamide**

En opérant comme dans l'exemple 2, mais en remplaçant le composé de l'exemple 1 par le composé de l'exemple 7, on obtient le produit du titre :

Rendement : 88 %
- Point de fusion : 154 - 156°C
- Caractéristiques spectrales en infra rouge :

νC=O : 1660 $cm^{-1}$
νNH : 3400 $cm^{-1}$
νOH = 3600 - 3300 $cm^{-1}$
νC=C : 1600 $cm^{-1}$
δNH : 1530 $cm^{-1}$

**Exemple 9 :**

**N-hexyl N-(4,6-diméthylpyridin-2-yl) (6-acétoxy-3,4-dihydro-2,5,7,8-tétraméthyl-2H[1]-benzopyran-2-yl) carboxamide**

En remplaçant dans le stade B de l'exemple 1 la N-(4,6-diméthylpyridin-2-yl) isobutylamine par la N-(4,6-diméthylpyridin-2-yl) hexylamine, on obtient le produit du titre.

**Exemple 10 :**

**N-hexyl N-(4,6-diméthylpyridin-2-yl) (6-hydroxy-3,4-dihydro-2,5,7,8-tétraméthyl-2H[1]-benzopyran-2-yl) carboxamide**

En opérant comme dans l'exemple 2, mais en remplaçant le composé de l'exemple 1 par le composé de l'exemple 9, on obtient le produit du titre.

- Point de Fusion : 114 - 115°C ;

**Exemple 11 :**

**N-cyclohexylméthyl N-(4,6-diméthylpyridin-2-yl) (6-acétoxy-3,4-dihydro-2,5,7,8-tétraméthyl-2H[1]-benzopyran-2-yl) carboxamide**

En remplaçant dans le stade B de l'exemple 1 la N-(4,6-diméthylpyridin-2-yl) isobutylamine par la N-(4,6-diméthylpyridin-2-yl) cyclohexylméthylamine, on obtient le produit du titre.

**Exemple 12 :**

**N-cyclohexylméthyl N-(4,6-diméthylpyridin-2-yl) (6-hydroxy-3,4-dihydro-2,5,7,8-tétraméthyl-2H[1]-benzopyran-2-yl) carboxamide**

En opérant comme dans l'exemple 2, mais en remplaçant le composé de l'exemple 1 par le composé de l'exemple 11, on obtient le produit du titre

**Exemple 13 :**

**N-phényl N-(butèn-3 yl) (6-acétoxy-3,4-dihydro-2,5,7,8-tétraméthyl-2H[1]-benzopyran-2-yl) carboxamide**

En remplaçant dans le stade B de l'exemple 1 la N-(4,6-diméthylpyridin-2-yl) isobutylamine par la N-(butèn-3 yl) aniline, on obtient le produit du titre.

**Exemple 14 :**

**N-phényl N-(butèn-3 yl) (6-hydroxy-3,4-dihydro-2,5,7,8-tétraméthyl-2H[1]-benzopyran-2-yl) carboxamide**

En opérant comme dans l'exemple 2, mais en remplaçant le composé de l'exemple 1 par le composé de l'exemple 13, on obtient le produit du titre.

**Exemple 15 :**

**N-furfuryl (6-acétoxy-3,4-dihydro-2,5,7,8-tétraméthyl-2H [1]-benzopyran-2-yl) carboxamide**

En remplaçant dans le stade B de l'exemple 1 la N-(4,6-diméthylpyridin-2-yl) isobutylamine par la furfurylamine, on obtient le produit du titre :

Rendement : 69,7 %

- Point de fusion : 127 - 128°C

**Exemple 16 :**

**N-furfuryl (6-hydroxy-3,4-dihydro-2,5,7,8-tétraméthyl-2H[1]-benzopyran-2-yl) carboxamide**

En opérant comme dans l'exemple 2, mais en remplaçant le composé de l'exemple 1 par le composé de l'exemple 15, on obtient le produit du titre :

Rendement : 80,8 %

- Point de fusion : 109 - 112°C
- Caractéristiques spectrales en infra rouge :

νC=O : 1655 $cm^{-1}$
νNH : 3420 $cm^{-1}$
νOH : 3550, 3350 $cm^{-1}$

**Exemple 17 :**

**N-(4-hydroxy-2,3-diméthylphényl) (6-acétoxy-3,4-dihydro-2,5,7,8-tétraméthyl-2H[1]-benzopyran-2-yl) carboxamide**

En remplaçant dans le stade B de l'exemple 1 la N-(4,6-diméthylpyridin-2-yl) isobutylamine par la 4-hydroxy-2,3-diméthylaniline, on obtient le produit du titre :

Rendement : 63,7 %

- Point de fusion : 230 - 232°C

**Exemple 18 :**

**N-(4-hydroxy-2,3-diméthylphényl) (6-hydroxy-3,4-dihydro-2,5,7,8-tétraméthyl-2H[1]-benzopyran-2-yl) carboxamide**

En opérant comme dans l'exemple 2, mais en remplaçant le composé de l'exemple 1 par le composé de l'exemple 17, on obtient le produit du titre :

Rendement : 71 %

- Point de fusion : 182 - 183°C
- Caractéristiques spectrales en infra rouge :

ν C=O (amide) : 1640 $cm^{-1}$
νNH : 3400 $cm^{-1}$
νOH : 3500, 3300 $cm^{-1}$
δNH : 1510 $cm^{-1}$

**Exemple 19 :**

**N-(5,7-diméthylnaphtyridin-2-yl) (6-acétoxy-3,4-dihydro-2,5,7,8-tétraméthyl-2H[1]-benzopyran-2-yl) carboxamide**

En remplaçant dans le stade B de l'exemple 1 la N-(4,6-diméthylpyridin-2-yl) isobutylamine par la 2-amino-5,7-diméthylnaphtyridine, on obtient le produit du titre :

Rendement : 76,8 %
- Point de fusion : 205 - 207°C

**Exemple 20 :**

**N-(5,7-diméthylnaphtyridin-2-yl) (6-hydroxy-3,4-dihydro-2,5,7,8-tétraméthyl-2H[1]-benzopyran-2-yl) carboxamide**

En opérant comme dans l'exemple 2, mais en remplaçant le composé de l'exemple 1 par le composé de l'exemple 19, on obtient le produit du titre :

Rendement : 86,5 %
- Point de fusion : 277 - 280°C
- Caractéristiques spectrales en infra rouge :

νC=O : 1690 $cm^{-1}$
νNH : 3390 $cm^{-1}$
νOH : 3500, 3200 $cm^{-1}$

**Exemple 21 :**

**N-cyclopropylméthyl N-(4,6-diméthylpyridin-2-yl) (6-acétoxy-3,4-dihydro-2,5,7,8-tétraméthyl-2H[1]-benzopyran-2-yl) carboxamide**

En remplaçant dans le stade B de l'exemple 1 la N-(4,6-diméthylpyridin-2-yl) isobutylamine par la N-(4,6-diméthylpyridin-2-yl) cyclopropylméthylamine et en changeant la durée de l'étape de chauffage au reflux de 3 heures en 24 heures, on obtient le produit du titre :

- Point de fusion : 105 - 106°C

**Exemple 22 :**

**N-cyclopropylméthyl N-(4,6-diméthylpyridin-2-yl) (6-hydroxy-3,4-dihydro-2,5,7,8-tétraméthyl-2H[1]-benzopyran-2-yl) carboxamide**

En opérant comme dans l'exemple 2, mais en remplaçant le composé de l'exemple 1 par le composé de l'exemple 21, on obtient le produit du titre :

Rendement : 95,8 %
- Point de fusion : 159 - 160°C
- Caractéristiques spectrales en infra rouge :

| | |
|---|---|
| νC=O | : 1630 $cm^{-1}$ |
| ν$CH_2$ (cyclopropane) | : 3080 $cm^{-1}$ |
| ν$CH_2$, $CH_3$ | : 2980, 2920, 2950 $cm^{-1}$ |
| νC=C, C=N | : 1605, 1555 $cm^{-1}$ |

**Exemple 23 :**

**N-(4-méthylquinoléin-2-yl) (6-acétoxy-3,4-dihydro-2,5,7,8-tétraméthyl-2H[1]-benzopyran-2-yl) carboxamide**

En remplaçant dans le stade B de l'exemple 1 la N-(4,6-diméthylpyridin-2-yl) isobutylamine par la 2-amino-4-méthylquinoléine et en changeant la durée de l'étape de chauffage au reflux de 3 heures en 24 heures, on obtient le produit du titre :

Rendement : 53,4 %
- Point de fusion : 205 - 207°C

**Exemple 24 :**

**N-(4-méthylquinoléin-2-yl) (6-hydroxy-3,4-dihydro-2,5,7,8-tétraméthyl-2H[1]-benzopyran-2-yl) carboxamide**

En opérant comme dans l'exemple 2, mais en remplaçant le composé de l'exemple 1 par le composé de l'exemple 23, on obtient le produit du titre :

Rendement : 91,7 %
- Point de fusion : 275 - 277°C
- Caractéristiques spectrales en infra rouge :

νC=O : 1700 $cm^{-1}$
νNH : 3380 $cm^{-1}$
νOH : 3600, 3300 $cm^{-1}$
νC=C, C=N : 1615, 1600, 1560 $cm^{-1}$
δ NH : 1520 $cm^{-1}$

**Exemple 25 :**

**N-phényl (6-acétoxy-3,4-dihydro-2,5,7,8-tétraméthyl-2H[1]-benzopyran-2-yl) carboxamide**

En remplaçant dans le stade B de l'exemple 1 la N-(4,6-diméthylpyridin-2-yl) isobutylamine par l'aniline, on obtient le produit du titre :

Rendement : 80,9 %
- Point de fusion : 104 - 105°C
- Caractéristiques spectrales en infra rouge :

νC=O (ester) : 1750 $cm^{-1}$
νC=O (amide) : 1685 $cm^{-1}$
νNH : 3400 $cm^{-1}$
νC=C : 1595 $cm^{-1}$
δNH : 1520 $cm^{-1}$

**Exemple 26 :**

**N-phényl (6-hydroxy-3,4-dihydro-2,5,7,8-tétraméthyl-2H[1]-benzopyran-2-yl) carboxamide**

En opérant comme dans l'exemple 2, mais en remplaçant le composé de l'exemple 1 par le composé de l'exemple 25, on obtient le produit du titre :

Rendement : 86 %
- Point de fusion : 107 - 109°C
- Caractéristiques spectrales en infra rouge :

νC=O : 1670 $cm^{-1}$
νNH : 3400 $cm^{-1}$
νOH : 3480 $cm^{-1}$
νC=C : 1600 $cm^{-1}$
δNH : 1520 $cm^{-1}$

**Exemple 27 :**

**(S)-(-)-N-phényl (6-hydroxy-3,4-dihydro-2,5,7,8-tétraméthyl-2H[1]-benzopyran-2-yl) carboxamide**

Pouvoir rotatoire $[\alpha]_D^{24}$ = - 262°.

**Exemple 28 :**

**(R)-(+)-N-phényl (6-hydroxy-3,4-dihydro-2,5,7,8-tétraméthyl-2H[1]-benzopyran-2-yl) carboxamide**

Pouvoir rotatoire $[\alpha]_D^{24}$ = + 256°.

**Exemple 29 :**

**N-(2-carboxy-4,5-diméthoxyphényl) (6-acétoxy-3,4-dihydro-2,5,7,8-tétraméthyl-2H[1]-benzopyran-2-yl) carboxamide**

En remplaçant dans le stade B de l'exemple 1 la N-(4,6-diméthylpyridin-2-yl) isobutylamine par la 2-carboxy-4,5-diméthoxyaniline, on obtient le produit du titre :

Rendement : 49,6 %
- Point de fusion : 254 - 256°C

**Exemple 30 :**

**N-(2-carboxy-4,5-diméthoxyphényl) (6-hydroxy-3,4-dihydro 2,5,7,8-tétraméthyl-2H[1]-benzopyran-2-yl) carboxamide**

En opérant comme dans l'exemple 2, mais en remplaçant le composé de l'exemple 1 par le composé de l'exemple 29 on obtient le produit du titre :

Rendement : 65,8 %

- Point de fusion : 244°C
- Caractéristiques spectrales en infra rouge :

| | |
|---|---|
| νC=O (amide) | : 1690 $cm^{-1}$ |
| νC=O (acide) | : 1660 $cm^{-1}$ |
| νNH | : 3360 $cm^{-1}$ |
| νOH | : 3200 $cm^{-1}$ |
| νC=C | : 1615, 1600 $cm^{-1}$ |
| δNH | : 1530 $cm^{-1}$ |

**Exemple 31 :**

**N-(3,5-dichloro-4-hydroxyphényl) (6-acétoxy-3,4-dihydro 2,5,7,8-tétraméthyl-2H[1]-benzopyran-2-yl) carboxamide**

En remplaçant dans le stade B de l'exemple 1 la N-(4,6-diméthylpyridin-2-yl) isobutylamine par la 3,5-dichloro-4-hydroxyaniline, on obtient le produit du titre :

Rendement : 48 %

- Point de fusion : 168°C

**Exemple 32 :**

**N-(3,5-dichloro-4-hydroxyphényl) (6-hydroxy-3,4-dihydro-2,5,7,8-tétraméthyl-2H[1]-benzopyran-2-yl) carboxamide**

En opérant comme dans l'exemple 2, mais en remplaçant le composé de l'exemple 1 par le composé de l'exemple 31 on obtient le produit du titre :

Rendement : 63 %

- Point de fusion : 208°C
- Caractéristiques spectrales en infra rouge :

| | |
|---|---|
| νC=O | : 1680 $cm^{-1}$ |
| νNH | : 3400 $cm^{-1}$ |
| νOH | : 1560, 1470 $cm^{-1}$ |
| νC=C | : 1570 $cm^{-1}$ |
| δNH | : 1520 $cm^{-1}$ |

**Exemple 33 :**

**N-(2-carboxy-4,6-diméthylphényl) (6-acétoxy-3,4-dihydro-2,5,7,8-tétraméthyl-2H[1]-benzopyran-2-yl) carboxamide**

En remplaçant dans le stade B de l'exemple 1 la N-(4,6-diméthylpyridin-2-yl) isobutylamine par la 2-carboxy-4,5-diméthylaniline, on obtient le produit du titre :

Rendement : 43,7 %

**Exemple 34 :**

**N-(2-carboxy-4,6-diméthylphényl) (6-hydroxy-3,4-dihydro-2,5,7,8-tétraméthyl-2H[1]-benzopyran-2-yl) carboxamide**

En opérant comme dans l'exemple 2, mais en remplaçant le composé de l'exemple 1 par le composé de l'exemple 33, on obtient le produit du titre :

Rendement : 71 %

- Point de fusion : 244°C
- Caractéristiques spectrales en infra rouge :

| | |
|---|---|
| νC=O (amide) | : 1710 $cm^{-1}$ |
| νC=O (acide) | : 1660 $cm^{-1}$ |
| νNH | : 3350 $cm^{-1}$ |
| νOH | : 3500 - 3200 $cm^{-1}$ |
| δNH | : 1505 $cm^{-1}$ |

**Exemple 35 :**

**N-(2,4,5-triméthylphényl) (6-acétoxy-3,4-dihydro-2,5,7,8-tétraméthyl-2H[1]-benzopyran-2-yl) carboxamide**

En remplaçant dans le stade B de l'exemple 1 la N-(4,6-diméthylpyridin-2-yl) isobutylamine par la 2,4,5-triméthylaniline, on obtient le produit du titre :

Rendement : 76,5 %
- Point de fusion : 160 - 162°C

**Exemple 36 :**

**N-(2,4,5-triméthylphényl) (6-hydroxy-3,4-dihydro-2,5,7,8-tétraméthyl-2H[1]-benzopyran-2-yl) carboxamide**

En opérant comme dans l'exemple 2, mais en remplaçant le composé de l'exemple 1 par le composé de l'exemple 35, on obtient le produit du titre :

Rendement : 64,5 %
- Point de fusion : 180 - 182°C
- Caractéristiques spectrales en infra rouge :

νC=O : 1670 $cm^{-1}$
νNH : 3410 $cm^{-1}$
νOH : 3480 $cm^{-1}$
νC=C : 1580 $cm^{-1}$
δNH : 1520 $cm^{-1}$

**Exemple 37 :**

**N-(2-méthylquinoléin-4-yl) (6-acétoxy-3,4-dihydro-2,5,7,8-tétraméthyl-2H[1]-benzopyran-2-yl) carboxamide**

En remplaçant dans le stade B de l'exemple 1 la N-(4,6-diméthylpyridin-2-yl) isobutylamine par la 4-amino-2-méthylquinoléine, on obtient le produit du titre :

Rendement : 52 %
- Point de fusion : 210°C

**Exemple 38 :**

**N-(2-méthylquinoléin-4-yl) (6-hydroxy-3,4-dihydro-2,5,7,8-tétraméthyl-2H[1]-benzopyran-2-yl) carboxamide**

En opérant comme dans l'exemple 2, mais en remplaçant le composé de l'exemple 1 par le composé de l'exemple 37, on obtient le produit du titre :

Rendement : 78,7 %
- Point de fusion : 273 - 275°C
- Caractéristiques spectrales en infra rouge :

νC=O : 1700 $cm^{-1}$
νNH : 3380 $cm^{-1}$
νC=C, C=N : 1620, 1600, 1560 $cm^{-1}$
δNH : 1520 $cm^{-1}$

**Exemple 39 :**

**1-oxa-2-oxo-3,8-diaza-8-((6-acétoxy-3,4-dihydro-2,5,7,8-tétraméthyl-2H[1]-benzopyran-2-yl)carbonyl) spiro[4,5]décane**

En remplaçant au stade B de l'exemple 1 la N-(4,6-diméthylpyridin-2-yl) isobutylamine par le 1-oxo-2-oxo-3,8-diaza-spiro[4,5]décane, on obtient le produit du titre :

Rendement : 62,3 %
- Point de fusion : 206°C

**Exemple 40 :**

**1-oxa-2-oxo-3,8-diaza-8-((6-hydroxy-3,4-dihydro-2,5,7,8-tétraméthyl-2H[1]-benzopyran-2-yl)carbonyl) spiro[4,5]décane**

En opérant comme dans l'exemple 2, mais en remplaçant le composé de l'exemple 1 par le composé de l'exemple 39, on obtient le produit du titre :

Rendement : 82,2 %

- Point de fusion : 218°C
- Caractéristiques spectrales en infra rouge :

νC=O (carbamate) : 1740 $cm^{-1}$
νC=O (amide) : 1620 $cm^{-1}$

**Exemple 41 :**

**N-isobutyl N-(4-méthylquinoléin-2-yl) (6-acétoxy-3,4-dihydro-2,5,7,8-tétraméthyl-2H[1]-benzopyran-2-yl) carboxamide**

En remplaçant dans le stade B de l'exemple 1 la N-(4,6-diméthylpyridin-2-yl) isobutylamine par la N-(4-méthylquinoléin-2-yl) isobutylamine et en changeant la durée de l'étape de chauffage au reflux de 3 heures en 24 heures, on obtient le produit du titre.

**Exemple 42 :**

**N-isobutyl N-(4-méthylquinoléin-2-yl) (6-hydroxy-3,4-dihydro-2,5,7,8-tétraméthyl-2H[1]-benzopyran-2-yl) carboxamide**

En opérant comme dans l'exemple 2, mais en remplaçant le composé de l'exemple 1 par le composé de l'exemple 41, on obtient le produit du titre.

**Exemple 43 :**

**N-(1-méthyl-β-carbolin-3-yl) (6-acétoxy-3,4-dihydro-2,5,7,8-tétraméthyl-2H[1]-benzopyran-2-yl) carboxamide**

En remplaçant dans le stade B de l'exemple 1 la N-(4,6-diméthylpyridin-2-yl) isobutylamine par la 3-amino-1-méthyl-β-carboline, on obtient le produit du titre.

**Exemple 44 :**

**N-(1-méthyl-β-carbolin-3-yl) (6-hydroxy-3,4-dihydro-2,5,7,8-tétraméthyl-2H[1]-benzopyran-2-yl) carboxamide**

En opérant comme dans l'exemple 2, mais en remplaçant le composé de l'exemple 1 par le composé de l'exemple 43, on obtient le produit du titre.

**Exemple 45 :**

**N-(4-chloronapht-1-yl) (6-acétoxy-3,4-dihydro-2,5,7,8-tétraméthyl-2H[1]-benzopyran-2-yl) carboxamide**

En remplaçant dans le stade B de l'exemple 1 la N-(4,6-diméthylpyridin-2-yl) isobutylamine par le 1-amino-4-chloronaphtalène, on obtient le produit du titre.

**Exemple 46 :**

**N-(4-chloronapht-1-yl) (6-hydroxy-3,4-dihydro-2,5,7,8-tétraméthyl-2H[1]-benzopyran-2-yl) carboxamide**

En opérant comme dans l'exemple 2, mais en remplaçant le composé de l'exemple 1 par le composé de l'exemple 45, on obtient le produit du titre.

**Exemple 47 :**

**N-(napht-2-yl) (6-acétoxy-3,4-dihydro-2,5,7,8-tétraméthyl-2H [1]-benzopyran-2-yl) carboxamide**

En remplaçant dans le stade B de l'exemple 1 la N-(4,6-diméthylpyridin-2-yl) isobutylamine par le 2-aminonaphtalène, on obtient le produit du titre.

**Exemple 48 :**

**N-(napht-2-yl) (6-hydroxy-3,4-dihydro-2,5,7, 8-tétraméthyl-2H [1]-benzopyran-2-yl) carboxamide**

En opérant comme dans l'exemple 2, mais en remplaçant le composé de l'exemple 1 par le composé de l'exemple 47, on obtient le produit du titre.

**Exemple 49 :**

**N-(3,4-dihydro-2H[1]-benzopyran-2-yl) (6-acétoxy-3,4-dihydro-2,5,7,8-tétraméthyl-2H[1]-benzopyran-2-yl) carboxamide**

En remplaçant dans le stade B de l'exemple 1 la N-(4,6-diméthylpyridin-2-yl) isobutylamine par le 2-amino-3,4-dihydro-2H[1]-benzopyrane, on obtient le produit du titre.

**Exemple 50 :**

**N-(3,4-dihydro-2H[1]-benzopyran-2-yl) (6-hydroxy-3,4-dihydro-2,5,7,8-tétraméthyl-2H[1]-benzopyran-2-yl) carboxamide**

En opérant comme dans l'exemple 2, mais en remplaçant le composé de l'exemple 1 par le composé de l'exemple 49, on obtient le produit du titre.

**Exemple 51 :**

**N-(3,4-dihydro-2H[1]-pyran-2-yl) (6-acétoxy-3,4-dihydro-2,5,7,8-tétraméthyl-2H[1]-benzopyran-2-yl) carboxamide**

En remplaçant dans le stade B de l'exemple 1 la N-(4,6-diméthylpyridin-2-yl) isobutylamine par la 2-amino-3,4 dihydro 2H[1]-pyrane, on obtient le produit du titre.

**Exemple 52 :**

**N-(3,4-dihydro-2H[1]-pyran-2-yl) (6-hydroxy-3,4-dihydro-2,5,7,8-tétraméthyl-2H[1]-benzopyran-2-yl) carboxamide**

En opérant comme dans l'exemple 2, mais en remplaçant le composé de l'exemple 1 par le composé de l'exemple 51, on obtient le produit du titre.

**Exemple 53 :**

**N-(isoquinoléin-5-yl) (6-acétoxy-3,4-dihydro-2,5,7,8-tétraméthyl-2H[1]-benzopyran-2-yl) carboxamide**

En remplaçant dans le stade B de l'exemple 1 la N-(4,6-diméthylpyridin-2-yl) isobutylamine par la 5-amino-isoquinoléine, on obtient le produit du titre.

**Exemple 54 :**

**N-(isoquinoléin-5-yl) (6-hydroxy-3,4-dihydro-2,5,7,8-tétraméthyl-2H[1]-benzopyran-2-yl) carboxamide**

En opérant comme dans l'exemple 2, mais en remplaçant le composé de l'exemple 1 par le composé de l'exemple 53, on obtient le produit du titre.

**Exemple 55 :**

**N-(thiazol-2-yl) (6-acétoxy-3,4-dihydro-2,5,7,8-tétraméthyl-2H[1]-benzopyran-2-yl) carboxamide**

En remplaçant dans le stade B de l'exemple 1 la N-(4,6-diméthylpyridin-2-yl) isobutylamine par le 2-aminothiazole, on obtient le produit du titre.

**Exemple 56 :**

**N-(thiazol-2-yl) (6-hydroxy-3,4-dihydro-2,5,7,8-tétraméthyl-2H[1]-benzopyran-2-yl) carboxamide**

En opérant comme dans l'exemple 2, mais en remplaçant le composé de l'exemple 1 par le composé de l'exemple 55, on obtient le produit du titre.

**Exemple 57 :**

**N-(3-méthylisothiazol-5-yl) (6-acétoxy-3,4-dihydro-2,5,7,8-tétraméthyl-2H[1]-benzopyran-2-yl) carboxamide**

En remplaçant dans le stade B de l'exemple 1 la N-(4,6-diméthylpyridin-2-yl) isobutylamine par le 5-amino-3-méthylisothiazole, on obtient le produit du titre.

**Exemple 58 :**

**N-(3-méthylisothiazol-5-yl) (6-hydroxy-3,4-dihydro-2,5,7,8-tétraméthyl-2H[1]-benzopyran-2-yl) carboxamide**

En opérant comme dans l'exemple 2, mais en remplaçant le composé de l'exemple 1 par le composé de l'exemple 57, on obtient le produit du titre.

**Exemple 59 :**

**N-{4-[(2,4-dioxo-5-thiazolidinyl)méthyl]phényl} (6-acétoxy-3,4-dihydro-2,5,7,8-tétraméthyl-2H[1]-benzopyran-2-yl) carboxamide**

En remplaçant dans le stade B de l'exemple 1 la N-(4,6-diméthylpyridin-2-yl) isobutylamine par la N-{4-[(2,4-dioxo-5-thiazolidinyl)méthyl]phényl}amine, on obtient le produit du titre

**Exemple 60 :**

**N-{4-[(2,4-dioxo-5-thiazolidinyl)méthyl]phényl} (6-hydroxy-3,4-dihydro-2,5,7,8-tétraméthyl-2H[1]-benzopyran-2-yl) carboxamide**

En opérant comme dans l'exemple 2, mais en remplaçant le composé de l'exemple 1 par le composé de l'exemple 59, on obtient le produit du titre.

- Point de fusion : 108 - 112°C
- Caractéristiques spectrales en infra rouge :

$\nu$N-H : 3400, 3200 $cm^{-1}$
$\nu$C = O : 1750, 1690 $cm^{-1}$

**Exemple 61 :**

**N-(5-méthylisoxazol-3-yl) (6-acétoxy-3,4-dihydro-2,5,7,8-tétraméthyl-2H[1]-benzopyran-2-yl) carboxamide**

En remplaçant dans le stade B de l'exemple 1 la N-(4,6-diméthylpyridin-2-yl) isobutylamine par le 3-amino-5-méthylisoxazole, on obtient le produit du titre.

**Exemple 62 :**

**N-(5-méthylisoxazol-3-yl) (6-hydroxy-3,4-dihydro-2,5,7,8-tétraméthyl-2H[1]-benzopyran-2-yl) carboxamide**

En opérant comme dans l'exemple 2, mais en remplaçant le composé de l'exemple 1 par le composé de l'exemple 61, on obtient le produit du titre.

**Exemple 63 :**

**N-((3-hydroxyisoxazol-5-yl)méthyl) (6-acétoxy-3,4-dihydro-2,5,7,8-tétraméthyl-2H[1]-benzopyran-2-yl) carboxamide**

En remplaçant dans le stade B de l'exemple 1 la N-(4,6-diméthylpyridin-2-yl) isobutylamine par le muscimol, on obtient le produit du titre.

**Exemple 64 :**

**N-((3-hydroxyisoxazol-5-yl)méthyl) (6-hydroxy-3,4-dihydro-2,5,7,8-tétraméthyl-2H[1]-benzopyran-2-yl) carboxamide**

En opérant comme dans l'exemple 2, mais en remplaçant le composé de l'exemple 1 par le composé de

l'exemple 63, on obtient le produit du titre.

**Exemple 65 :**

**N-((thién-2-yl)méthyl) (6-acétoxy-3,4-dihydro-2,5,7,8-tétraméthyl-2H[1]-benzopyran-2-yl) carboxamide**

En remplaçant dans le stade B de l'exemple 1 la N-(4,6-diméthylpyridin-2-yl) isobutylamine par la 2-thiophèneméthylamine, on obtient le produit du titre.

**Exemple 66 :**

**N-((thién-2-yl)méthyl) (6-hydroxy-3,4-dihydro-2,5,7,8-tétraméthyl-2H[1]-benzopyran-2-yl) carboxamide**

En opérant comme dans l'exemple 2, mais en remplaçant le composé de l'exemple 1 par le composé de l'exemple 65, on obtient le produit du titre.

**Exemple 67 :**

**N-(benzofuran-2-yl) (6-acétoxy-3,4-dihydro-2,5,7,8-tétraméthyl-2H[1]-benzopyran-2-yl) carboxamide**

En remplaçant dans le stade B de l'exemple 1 la N-(4,6-diméthylpyridin-2-yl) isobutylamine par le 2-aminobenzofurane, on obtient le produit du titre.

**Exemple 68 :**

**N-(benzofuran-2-yl) (6-hydroxy-3,4-dihydro-2,5,7,8-tétraméthyl-2H[1]-benzopyran-2-yl) carboxamide**

En opérant comme dans l'exemple 2, mais en remplaçant le composé de l'exemple 1 par le composé de l'exemple 67, on obtient le produit du titre.

**Exemple 69 :**

**N-butyl N-phényl (6-acétoxy-3,4-dihydro-2,5,7,8-tétraméthyl-2H[1]-benzopyran-2-yl) carboxamide**

En remplaçant dans le stade B de l'exemple 1 la N-(4,6-diméthylpyridin-2-yl) isobutylamine par la N-butylaniline, on obtient le produit du titre.

- Rendement : 50 %
- Point de fusion : 135°C

**Exemple 70 :**

**N-butyl N-phényl (6-hydroxy-3,4-dihydro-2,5,7,8-tétraméthyl-2H[1]-benzopyran-2-yl) carboxamide**

En opérant comme dans l'exemple 2, mais en remplaçant le composé de l'exemple 1 par le composé de l'exemple 69, on obtient le produit du titre.

**Exemple 71 :**

**N-(napht-1-yl) N-phényl (6-acétoxy-3,4-dihydro-2, 5, 7, 8-tétraméthyl -2H[1]-benzopyran-2-yl) carboxamide**

En remplaçant dans le stade B de l'exemple 1 la N-(4,6-diméthylpyridin-2-yl) isobutylamine par la N-phényl (napht-1-yl)amine, on obtient le produit du titre.

**Exemple 72 :**

**N-(napht-1-yl) N-phényl (6-hydroxy-3,4-dihydro-2,5,7,8-tétraméthyl -2H[1]-benzopyran-2-yl) carboxamide**

En opérant comme dans l'exemple 2, mais en remplaçant le composé de l'exemple 1 par le composé de l'exemple 71, on obtient le produit du titre.

**Exemple 73 :**

**N-allyl N-phényl (6-acétoxy-3,4-dihydro-2,5,7,8-tétraméthyl-2H[1]-benzopyran-2-yl) carboxamide**

En remplaçant dans le stade B de l'exemple 1 la N-(4,6-diméthylpyridin-2-yl) isobutylamine par la N-allylaniline, on obtient le produit du titre.

**Exemple 74 :**

**N-allyl N-phényl (6-hydroxy-3,4-dihydro-2,5,7,8-tétraméthyl-2H[1]-benzopyran-2-yl) carboxamide**

En opérant comme dans l'exemple 2, mais en remplaçant le composé de l'exemple 1 par le composé de l'exemple 73, on obtient le produit du titre.

**Exemple 75 :**

**N-cyclopropyl N-phényl (6-acétoxy-3,4-dihydro-2,5,7,8-tétraméthyl-2H[1]-benzopyran-2-yl) carboxamide**

En remplaçant dans le stade B de l'exemple 1 la N-(4,6-diméthylpyridin-2-yl) isobutylamine par la N-cyclopropylaniline, on obtient le produit du titre.

**Exemple 76 :**

**N-cyclopropyl N-phényl (6-hydroxy-3,4-dihydro-2,5,7,8-tétraméthyl-2H[1]-benzopyran-2-yl) carboxamide**

En opérant comme dans l'exemple 2, mais en remplaçant le composé de l'exemple 1 par le composé de l'exemple 75, on obtient le produit du titre.

**Exemples 77 à 102 :**

De même, en suivant les exemples 1 et 2, et en utilisant les amines appropriées, on obtient les produits suivants :

**Exemple 77 :**

**N-éthyl N-(4-méthylquinoléin-2-yl) (6-acétoxy-3,4-dihydro-2,5,7,8-tétraméthyl-2H[1]-benzopyran-2-yl) carboxamide**

**Exemple 78 :**

**N-éthyl N-(4-méthylquinoléin-2-yl) (6-hydroxy-3,4-dihydro-2,5,7,8-tétraméthyl-2H[1]-benzopyran-2-yl) carboxamide**

**Exemple 79 :**

**N-(butèn-3 yl) N-(4-méthylquinoléin-2-yl) (6-acétoxy-3,4-dihydro-2,5,7,8-tétraméthyl-2H[1]-benzopyran-2-yl) carboxamide**

**Exemple 80 :**

**N-(butèn-3 yl) N-(4-méthylquinoléin-2-yl) (6-hydroxy-3,4-dihydro-2,5,7,8-tétraméthyl-2H[1]-benzopyran-2-yl) carboxamide**

**Exemple 81 :**

**N-phényl N-(4,6-diméthylpyridin-2-yl) (6-acétoxy-3,4-dihydro-2,5,7,8-tétraméthyl-2H[1]-benzopyran-2-yl) carboxamide**

**Exemple 82 :**

**N-phényl N-(4,6-diméthylpyridin-2-yl) (6-hydroxy-3,4-dihydro-2,5,7,8-tétraméthyl-2H[1]-benzopyran-2-yl) carboxamide**

**Exemple 83 :**

**N-(éthoxyprop-1-yl) (6-acétoxy-3,4-dihydro-2,5,7,8-tétraméthyl-2H[1]-benzopyran-2-yl) carboxamide**

**Exemple 84 :**

**N-(éthoxyprop-1-yl) (6-hydroxy-3,4-dihydro-2,5,7,8-tétraméthyl-2H[1]-benzopyran-2-yl) carboxamide**

**Exemple 85 :**

**N-(méthoxyéthyl) (6-acétoxy-3,4-dihydro-2,5,7,8-tétraméthyl-2H[1]-benzopyran-2-yl) carboxamide**

**Exemple 86 :**

**N-(méthoxyéthyl) (6-hydroxy-3,4-dihydro-2,5,7,8-tétraméthyl-2H[1]-benzopyran-2-yl) carboxamide**
- Point de fusion : 115 - 116°C

**Exemple 87 :**

**N-phényl N-éthoxycarbonylméthyl (6-acétoxy-3,4-dihydro-2,5,7,8-tétraméthyl-2H[1]-benzopyran-2-yl) carboxamide**

**Exemple 88 :**

**N-phényl N-éthoxycarbonylméthyl (6-hydroxy-3,4-dihydro-2,5,7,8-tétraméthyl-2H[1]-benzopyran-2-yl) carboxamide**

**Exemple 89 :**

**1-((6-acétoxy-3,4-dihydro-2,5,7,8-tétraméthyl-2H[1]-benzopyran-2-yl) carbonyl) 5,5-diméthylbiguanide**

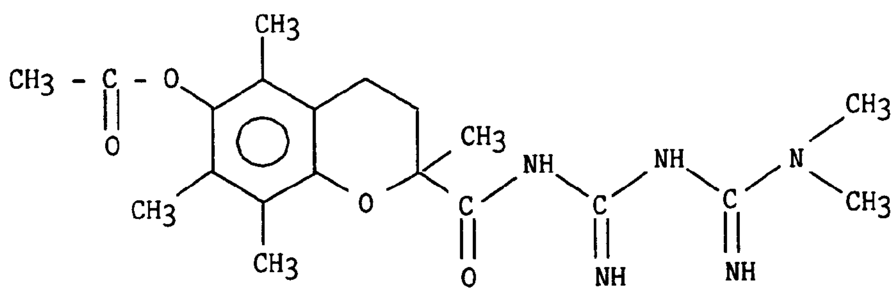

(exemple 89)

**Exemple 90 :**

**1-((6-hydroxy-3,4-dihydro-2,5,7,8-tétraméthyl-2H[1]-benzopyran-2-yl) carbonyl) 5,5-diméthylbiguanide**

**Exemple 91 :**

**N-guanidino (6-acétoxy-3,4-dihydro-2,5,7,8-tétraméthyl-2H[1]-benzopyran-2-yl) carboxamide**

**Exemple 92 :**

**N-guanidino (6-hydroxy-3,4-dihydro-2,5,7,8-tétraméthyl-2H[1]-benzopyran-2-yl) carboxamide**

**Exemple 93 :**

**1-((6-acétoxy-3,4-dihydro-2,5,7,8-tétraméthyl-2H[1]-benzopyran-2-yl) carbonyl) 3,3-diméthylguanidine**

**Exemple 94 :**

**1-((6-hydroxy-3,4-dihydro-2,5,7,8-tétraméthyl-2H[1]-benzopyran-2-yl) carbonyl) 3,3-diméthylguanidine**

**Exemple 95 :**

**N-(4-amino-5-(3,4,5-triméthoxybenzyl)-pyrimidin-2-yl) (6-acétoxy-3,4-dihydro 2,5,7,8-tétraméthyl-2H [1]-benzopyran-2-yl) carboxamide**

**Exemple 96 :**

**N-(4-amino-5-(3,4,5-triméthoxybenzyl)-pyrimidin-2-yl) (6-hydroxy 3,4-dihydro 2,5,7,8-tétraméthyl-2H [1]-benzopyran-2-yl) carboxamide**

**Exemple 97 :**

**N-(4-amino-5-(4-chlorophényl)-6-éthylpyrimidin-2-yl) (6-acétoxy-3,4-dihydro-2,5,7,8-tétraméthyl-2H[1]-benzopyran-2-yl) carboxamide**

**Exemple 98 :**

**N-(4-amino-5-(4-chlorophényl)-6-éthylpyrimidin-2-yl) (6-hydroxy-3,4-dihydro-2,5,7,8-tétraméthyl-2H[1]-benzopyran-2-yl) carboxamide**

**Exemple 99 :**

**N-{4-[(2-oxo-1,2,3,5-oxathiadiazol-4-yl)méthyl]phényl} (6-acétoxy-3,4-dihydro-2,5,7,8-tétraméthyl-2H[1]-benzopyran-2-yl) carboxamide**

**Exemple 100 :**

**N-{4-[(2-oxo-1,2,3,5-oxathiadiazol-4-yl)méthyl]phényl} (6-hydroxy-3,4-dihydro-2,5,7,8-tétraméthyl-2H[1]-benzopyran-2-yl) carboxamide**

**Exemple 101 :**

**N-(6-chlorohexyl) (6-acétoxy-3,4-dihydro-2,5,7,8-tétraméthyl-2H[1]-benzopyran-2-yl) carboxamide**

**Exemple 102 :**

**N-(6-chlorohexyl) (6-hydroxy-3,4-dihydro-2,5,7,8-tétraméthyl-2H[1]-benzopyran-2-yl) carboxamide**

**Exemple 103 :**

**N-(1,3-dihydroxy-2-méthylprop-2-yl) (6-acétoxy-3,4-dihydro-2,5,7,8-tétraméthyl-2H[1]-benzopyran-2-yl) carboxamide**

En remplaçant dans le stade B de l'exemple 1 la N-(4,6-diméthylpyridin-2-yl) isobutylamine par le 2-amino-2-méthyl-1,3-propanediol, on obtient le produit du titre :

Rendement : 60,32 %

- Point de fusion : 117 - 119°C
- Caractéristiques spectrales en infra rouge :

| | |
|---|---|
| νC=O (ester) | : 1720 $cm^{-1}$ |
| νC=O (amide) | : 1660 $cm^{-1}$ |
| νNH | : 3420 $cm^{-1}$ |
| δNH | : 1515 $cm^{-1}$ |

**Exemple 104 :**

**N-(2,2,5-triméthyl-1,3-dioxan-5-yl) (6-acétoxy-3,4-dihydro-2,5,7,8-tétraméthyl-2H[1]-benzopyran-2-yl) carboxamide**

Dissoudre 3 grammes (7,9 mmoles) du composé de l'exemple 103 dans 45 $cm^3$ de 2,2-diméthoxypropane, ajouter 15 $cm^3$ de diméthylformamide anhydre puis 60 milligrammes d'acide paratoluène sulfonique. Chauffer

au reflux pendant une heure. Evaporer l'excès de 2,2-diméthoxypropane sous vide. Diluer le mélange résiduel à l'eau. Extraire le produit avec du dichlorométhane. Laver la phase organique avec une solution de bicarbonate de sodium puis à l'eau.

Purifier l'amide par passage sur colonne de gel de silice en éluant avec de l'éther éthylique. On obtient le produit du titre :

Rendement : 93,5 %
- Point de fusion : 97 - 99°C

**Exemple 105 :**

**N-(2,2,5-triméthyl-1,3-dioxan-5-yl) (6-hydroxy-3,4-dihydro-2,5,7,8-tétraméthyl-2H[1]-benzopyran-2-yl) carboxamide**

En opérant comme dans l'exemple 2, mais en remplaçant le composé de l'exemple 1 par le composé de l'exemple 104, on obtient le produit du titre :

Rendement : 87 %
- Point de fusion : 153 - 154°C
- Caractéristiques spectrales en infra rouge :

| | |
|---|---|
| νC=O (amide) | : 1650 $cm^{-1}$ |
| νNH | : 3410 $cm^{-1}$ |
| νOH | : 3500 - 3300 $cm^{-1}$ |
| δNH | : 1515 $cm^{-1}$ |

**Exemple 106 :**

**N-(4,6-diméthylpyridin-2-yl) (6-méthoxyméthoxy-3,4-dihydro-2,5,7,8-tétraméthyl-2H[1]-benzopyran-2-yl) carboxamide**

**STADE A :**

**Acide (6-méthoxyméthoxy-3,4-dihydro-2,5,7,8-tétraméthyl-2H[1]-benzopyran-2-yl) carboxylique**

Dissoudre 50 grammes (0,2 mole) d'acide (6-hydroxy-3,4-dihydro-2,5,7,8-tétraméthyl-2H[1]-benzopyran-2-yl) carboxylique dans 150 $cm^3$ de pyridine anhydre. Ajouter goutte à goutte 16,34 $cm^3$ (0,2 mole) de bromure d'oxyde de méthyle. Agiter pendant 2 heures puis verser la solution sur de la glace. Acidifier avec de l'acide acétique, puis extraire avec du chlorure de méthylène. Laver la phase organique à l'eau et sécher sur sulfate de sodium anhydre, puis purifier le produit du titre par passage sur colonne de gel de silice en éluant avec du chlorure de méthylène.

**STADE B :**

**N-(4,6-diméthylpyridin-2-yl) (6-méthoxyméthoxy-3,4-dihydro-2,5,7,8-tétraméthyl-2H[1]-benzopyran-2-yl) carboxamide**

Dissoudre 5,4 grammes (18,47 mmoles) du composé au stade A de l'exemple 106 dans 30 $cm^3$ de benzène anhydre, ajouter 2,2 $cm^3$ (27,41 mmoles) de chlorure de thionyle, chauffer au reflux pendant 3 heures, chasser le solvant sous vide en éliminant l'excès de chlorure de thionyle. Dissoudre le chlorure d'acide ainsi obtenu dans 30 $cm^3$ de dichloroéthane. Dans un autre récipient, dissoudre 2,26 grammes (18,5 mmoles) de 2-amino-4,6-diméthylpyridine dans 20 $cm^3$ de dichloroéthane, ajouter 7,7 $cm^3$ de triéthylamine et verser goutte à goutte dans ce mélange la solution de chlorure d'acide précédente. Après 8 heures d'agitation, évaporer le solvant sous vide, reprendre le résidu par 30 $cm^3$ d'eau, neutraliser avec une solution de $NaHCO_3$, extraire par du chlorure de méthylène, laver la phase organique à l'eau, puis la sécher sur sulfate de sodium. Après évaporation du solvant, purifier par chromatographie sur gel de silice en éluant avec du chlorure de méthylène.

On obtient le produit du titre :

Rendement : 60,2 %
- Point de fusion : 75 - 76°C
- Caractéristiques spectrales en infra rouge :

| | |
|---|---|
| νC=O | : 1690 $cm^{-1}$ |
| νNH | : 3410 $cm^{-1}$ |
| $νCH_2$, $CH_3$ | : 2980, 2940, 2860 $cm^{-1}$ |
| νC=C,C=N | : 1610, 1570 $cm^{-1}$ |

δNH : 1520 $cm^{-1}$

**Exemple 107 :**

**N-(4,6-diméthylpyridin-2-yl) (6-éthoxycarbonylméthoxy-3,4-dihydro-2,5,7,8-tétraméthyl-2H[1]-benzopyran-2-yl) carboxamide**

En procédant comme dans l'exemple 106, mais en remplaçant au stade A de l'exemple 106 le bromure d'oxyde de méthyle par le bromacétate d'éthyle, on obtient le produit du titre :

Rendement : 69,5 %

- Point de fusion : 128 - 129°C
- Caractéristiques spectrales en infra rouge :

| | |
|---|---|
| νC=O (ester) | : 1760 $cm^{-1}$ |
| νC=O (amide) | : 1685 $cm^{-1}$ |
| νNH | : 3400 $cm^{-1}$ |
| $\nu CH_2$, $CH_3$ | : 2980, 2920, 2860 $cm^{-1}$ |
| νC= C,C = N | : 1620, 1570 $cm^{-1}$ |
| δNH | : 1510 $cm^{-1}$ |

**Exemple 108 :**

**N-(4,6-diméthylpyridin-2-yl) (6-carboxyméthoxy-3,4-dihydro-2,5,7,8-tétraméthyl-2H[1]-benzopyran-2-yl) carboxamide**

Dissoudre 1,77 grammes (4 mmoles) du composé de l'exemple 107 dans 40 $cm^3$ d'éthanol. Ajouter goutte à goutte 4 $cm^3$ d'hydroxyde de sodium 2N. Agiter pendant 2 heures, puis diluer le mélange réactionnel avec 60 $cm^3$ d'eau, acidifier avec de l'acide acétique. Filtrer, laver à l'eau puis sécher. On obtient 1,55 grammes du produit du titre :

- Point de fusion : 230 - 231°C
- Caractéristiques spectrales en infra rouge :

| | |
|---|---|
| νC=O (acide) | : 1730 $cm^{-1}$ |
| νC=O (amide) | : 1700 $cm^{-1}$ |
| νNH | : 3400 $cm^{-1}$ |
| $\nu CH_2$, $CH_3$ | : 2980, 2920, 2860 $cm^{-1}$ |
| νC = C, C = N | : 1620, 1570 $cm^{-1}$ |
| δNH | : 1520 $cm^{-1}$ |

**Exemple 109 :**

**N-(4,6-diméthylpyridin-2-yl) (6-éthoxyéthoxy-3,4-dihydro-2,5,7,8-tétraméthyl-2H[1]-benzopyran-2-yl) carboxamide**

En procédant comme dans l'exemple 106, mais en remplaçant dans le stade A de l'exemple 106 le bromure d'oxyde de méthyle par le bromure d'oxyde d'éthyle, on obtient le produit du titre.

- Point de fusion : 96 - 98°C

**Exemple 110 :**

**N-(4,6-diméthylpyridin-2-yl) (6-éthoxycarbonyloxy-3,4-dihydro-2,5,7,8-tétraméthyl-2H[1]-benzopyran-2-yl) carboxamide**

En procédant comme dans l'exemple 106, mais en remplaçant dans le stade A de l'exemple 106 le bromure d'oxyde de méthyle par le bromate d'éthyle, on obtient le produit du titre :

Rendement : 63 %

- Point de fusion : < 50°C
- Caractéristiques spectrales en infra rouge :

| | |
|---|---|
| νC=O (ester) | : 1760 $cm^{-1}$ |
| νC=O (amide) | : 1700 $cm^{-1}$ |
| νNH | : 3410 $cm^{-1}$ |
| νCH ($CH_2$, $CH_3$) | : 2980, 2940, 2880 $cm^{-1}$ |
| ν C=C, C=N | : 1610, 1570 $cm^{-1}$ |
| δNH | : 1520 $cm^{-1}$ |

**Exemple 111 :**

**N-(4-méthylquinoléin-2-yl) (6-méthoxyméthoxy-3,4-dihydro-2,5,7,8-tétraméthyl-2H[1]-benzopyran-2-yl) carboxamide**

En remplaçant dans le stade B de l'exemple 106 la 2-amino-4,6-diméthylpyridine par la 2-amino-4-méthylquinoléine, on obtient le produit du titre.

**Exemples 112 à 115 :**

De même, en remplaçant dans le stade B de l'exemple 106 la 2-amino-4 ,6-diméthylpyridine par la 2-amino-4-méthylquinoléine et en opérant comme dans les exemples 108 à 110, on obtient les composés des exemples suivants :

**Exemple 112 :**

**N-(4-méthylquinoléin-2-yl) (6-éthoxycarbonylméthoxy-3,4-dihydro-2,5,7,8-tétraméthyl-2H[1]-benzopyran-2-yl) carboxamide**

**Exemple 113 :**

**N-(4-méthylquinoléin-2-yl) (6-carboxyméthoxy-3,4-dihydro-2,5,7,8-tétraméthyl-2H[1]-benzopyran-2-yl) carboxamide**

**Exemple 114 :**

**N-(4-méthylquinoléin-2-yl) (6-éthoxyéthoxy-3,4-dihydro-2,5,7,8-tétraméthyl-2H[1]-benzopyran-2-yl) carboxamide**

**Exemple 115 :**

**N-(4-méthylquinoléin-2-yl) (6-éthoxycarbonyloxy-3,4-dihydro-2,5,7,8-tétraméthyl-2H[1]-benzopyran-2-yl) carboxamide**

**Exemple 116 :**

**N-phényl (6-méthoxyméthoxy-3,4-dihydro-2,5,7,8-tétraméthyl-2H[1]-benzopyran-2-yl) carboxamide**

En remplaçant dans le stade B de l'exemple 106 la 2-amino-4,6-diméthylpyridine par l'aniline, on obtient le produit du titre.

**Exemples 117 à 120 :**

De même, en remplaçant dans le stade B de l'exemple 106 la 2-amino-4,6-diméthylpyridine par l'aniline et en suivant les opérations décrites dans les exemples 107 à 110, on obtient les produits des exemples suivants :

**Exemple 117 :**

**N-phényl (6-éthoxycarbonylméthoxy-3,4-dihydro-2,5,7,8-tétraméthyl-2H[1]-benzopyran-2-yl) carboxamide**

**Exemple 118 :**

**N-phényl (6-carboxyméthoxy-3,4-dihydro-2,5,7,8-tétraméthyl-2H[1]-benzopyran-2-yl) carboxamide**

**Exemple 119 :**

**N-phényl (6-éthoxyéthoxy-3,4-dihydro-2,5,7,8-tétraméthyl-2H[1]-benzopyran-2-yl) carboxamide**

**Exemple 120 :**

**N-phényl (6-éthoxycarbonyloxy-3,4-dihydro-2,5,7,8-tétraméthyl-2H[1]-benzopyran-2-yl) carboxamide**

**Exemple 121 :**

**N-(4-méthylquinoléin-2-yl) ((6-hydroxy-3,4-dihydro-2,5,7,8-tétraméthyl-2H[1]-benzopyran-2-yl) méthyl) amine**

Dissoudre 2,2 grammes (5,6 mmoles) du composé de l'exemple 24 dans 120 $cm^3$ de tétrahydrofurane anhydre, ajouter 0,94 grammes d'hydrure de lithium et d'aluminium, puis chauffer à reflux pendant 3 heures.

Après refroidissement, verser le mélange sur de la glace, filtrer et extraire le produit par du chloroforme chaud. Sécher la phase organique sur sulfate de sodium. Après élimination du solvant, triturer le résidu huileux pour obtenir le produit du titre :

- Caractéristiques spectrales en infra rouge :

| | |
|---|---|
| νNH | : 3380 $cm^{-1}$ |
| νCH | : 2970, 2920 $cm^{-1}$ |
| νOH | : 3600, 3300 $cm^{-1}$ |
| νC= C,C = N | : 1615, 1600, 1560 $cm^{-1}$ |

- Point de Fusion : 196 - 198°C

**Exemple 122 :**

**N-(4-méthylquinoléin-2-yl) ((6-acétoxy-3,4-dihydro-2,5,7,8-tétraméthyl-2H[1]-benzopyran-2-yl) méthyl) amine**

En opérant comme au stade A de l'exemple 1, mais en remplaçant l'acide (6-hydroxy-3,4-dihydro-2,5,7,8-tétraméthyl-2H[1]-benzopyran-2-yl) carboxylique par le composé de l'exemple 121, on obtient le produit du titre.

**Exemple 123 :**

**N-isobutyl N-(4-méthylquinoléin-2-yl) ((6-hydroxy-3,4-dihydro-2,5,7,8-tétraméthyl-2H[1]-benzopyran-2-yl)méthyl) amine**

En procédant comme dans l'exemple 121, mais en remplaçant le composé de l'exemple 24 par le composé de l'exemple 42, on obtient le produit du titre.

**Exemple 124 :**

**N-isobutyl N-(4-méthylquinoléin-2-yl) ((6-acétoxy-3,4-dihydro -2,5,7,8-tétraméthyl-2H[1]-benzopyran-2-yl) méthyl) amine**

En opérant comme au stade A de l'exemple 1, mais en remplaçant l'acide (6-hydroxy-3,4-dihydro-2,5,7,8-tétraméthyl-2H[1]-benzopyran-2-yl) carboxylique par le composé de l'exemple 123, on obtient le produit du titre.

**Exemple 125 :**

**N-(4,6-diméthylpyridin-2-yl) ((6-éthoxyéthoxy-3,4-dihydro-25,7,8-tétraméthyl-2H[1]-benzopyran-2-yl) méthyl) amine**

En procédant comme dans l'exemple 121, mais en remplaçant le composé de l'exemple 24 par le composé de l'exemple 109, on obtient le produit du titre.

**Exemples 126 à 147** :

De même, en procédant comme dans l'exemple 121 mais en remplaçant le composé de l'exemple 24 par le composé de l'exemple 1, 2, 3, 4, 5, 6, 21, 22, 55, 56, 69, 70, 71, 72, 73, 74, 104, 105, 106, 107, 108, puis 110, on obtient respectivement les produits des titres suivants :

**Exemple 126 :**

**N-isobutyl N-(4,6-diméthylpyridin-2-yl) ((6-acétoxy-3,4-dihydro-2,5,7,8-tétraméthyl-2H[1]-benzopyran-2-yl) méthyl) amine**

**Exemple 127 :**

**N-isobutyl N-(4,6-diméthylpyridin-2-yl) ((6-hydroxy-3,4-dihydro-2,5,7,8-tétraméthyl-2H[1]-benzopyran-2-yl) méthyl) amine**

**Exemple 128 :**

**N-(2,4,6-triméthylphényl) ((6-acétoxy-3,4-dihydro-2,5,7,8-tétraméthyl-2H[1]-benzopyran-2-yl) méthyl) amine**

**Exemple 129 :**

**N-(2,4,6-triméthylphényl) ((6-hydroxy-3,4-dihydro-2,5,7,8-tétraméthyl-2H[1]-benzopyran-2-yl) méthyl) amine**

**Exemple 130 :**

**N-butyl N-(4,6-diméthylpyridin-2-yl) ((6-acétoxy-3,4-dihydro-2,5,7,8-tétraméthyl-2H[1]-benzopyran-2-yl) méthyl) amine**

**Exemple 131 :**

**N-butyl N-(4,6-diméthylpyridin-2-yl) ((6-hydroxy-3,4-dihydro-2,5,7,8-tétraméthyl-2H[1]-benzopyran-2-yl) méthyl) amine**

**Exemple 132 :**

**N-cyclopropylméthyl N-(4,6-diméthylpyridin-2-yl) ((6-acétoxy-3,4-dihydro-2,5,7,8-tétraméthyl-2H[1]-benzopyran-2-yl) méthyl) amine**

**Exemple 133 :**

**N-cyclopropylméthyl N-(4,6-diméthylpyridin-2-yl) ((6-hydroxy-3,4-dihydro-2,5,7,8-tétraméthyl-2H[1]-benzopyran-2-yl) méthyl) amine**

**Exemple 134 :**

**N-(thiazol-2-yl) ((6-acétoxy-3,4-dihydro-2,5,7,8-tétraméthyl-2H[1]-benzopyran-2-yl) méthyl) amine**

**Exemple 135 :**

**N-(thiazol-2-yl) ((6-hydroxy-3,4-dihydro-2,5,7,8-tétraméthyl-2H[1]-benzopyran-2-yl) méthyl) amine**

**Exemple 136 :**

**N-butyl N-phényl ((6-acétoxy-3,4-dihydro-2,5,7,8-tétraméthyl-2H[1]-benzopyran-2-yl) méthyl) amine**

**Exemple 137 :**

**N-butyl N-phényl ((6-hydroxy-3,4-dihydro-2,5,7,8-tétraméthyl-2H[1]-benzopyran-2-yl) méthyl) amine**

**Exemple 138 :**

**N-(napht-1-yl) N-phényl ((6-acétoxy-3,4-dihydro-2,5,7,8-tétraméthyl-2H[1]-benzopyran-2-yl) méthyl) amine**

**Exemple 139 :**

**N-(napht-1-yl) N-phényl ((6-hydroxy-3,4-dihydro-2,5,7,8-tétraméthyl-2H[1]-benzopyran-2-yl) méthyl) amine**

**Exemple 140 :**

**N-allyl N-phényl ((6-acétoxy-3,4-dihydro-2,5,7,8-tétraméthyl-2H[1]-benzopyran-2-yl) méthyl) amine**

**Exemple 141 :**

**N-allyl N-phényl ((6-hydroxy-3,4-dihydro-2,5,7,8-tétraméthyl-2H[1]-benzopyran-2-yl) méthyl) amine**

**Exemple 142 :**

**N-(2,2,5-triméthyl-1,3-dioxan-5-yl) ((6-acétoxy-3,4-dihydro-2,5,7,8-tétraméthyl-2H[1]-benzopyran-2-yl) méthyl) amine**

**Exemple 143 :**

**N-(2,2,5-triméthyl-1,3-dioxan-5-yl) ((6-hydroxy-3,4-dihydro-2,5,7,8-tétraméthyl-2H[1]-benzopyran-2-yl) méthyl) amine**

**Exemple 144 :**

**N-(4,6-diméthylpyridin-2-yl) ((6-méthoxyméthoxy-3,4-dihydro-2,5,7,8-tétraméthyl-2H[1]-benzopyran-2-yl) méthyl) amine**

**Exemple 145 :**

**N-(4,6-diméthylpyridin-2-yl) ((6-éthoxycarbonylméthoxy-3,4-dihydro-2,5,7,8-tétraméthyl-2H[1]-benzopyran-2-yl) méthyl) amine**

**Exemple 146 :**

**N-(4,6-diméthylpyridin-2-yl) ((6-carboxyméthoxy-3,4-dihydro-2,5,7,8-tétraméthyl-2H[1]-benzopyran-2-yl) méthyl) amine**

**Exemple 147 :**

**N-(4,6-diméthylpyridin-2-yl) ((6-éthoxycarbonyloxy-3,4-dihydro-2,5,7,8-tétraméthyl-2H[1]-benzopyran-2-yl) méthyl) amine**

**Exemple 148 :**

**N-(4-méthylquinoléin-2-yl) 2-(6-acétoxy-3,4-dihydro-2,5,7,8-tétraméthyl-2H[1]-benzopyran-2-yl) acétamide**

En procédant comme dans l'exemple 1, mais en remplaçant au stade A de l'exemple 1 l'acide (6-hydroxy-3,4-dihydro-2,5,7,8-tétraméthyl-2H[1]-benzopyran-2-yl) carboxylique par l'acide (6-hydroxy-3,4-dihydro-2,5,7,8-tétraméthyl-2H[1]-benzopyran-2-yl) acétique et en remplaçant au stade B de l'exemple 1 la N(4,6-diméthylpyridin-2-yl) isobutylamine par le 2-amino-4-méthylquinoléine, ainsi qu'en remplaçant au même stade B de l'exemple 1 l'étape de chauffage au reflux, après le mélange de la solution du chlorure d'acide et de l'amine, par une agitation de 24 heures à la température de 20°C, on obtient le produit du titre.

- Caractéristiques spectrales en infra rouge :

| | |
|---|---|
| νC=O (ester) | : 1760 $cm^{-1}$ |
| νC=O (amide) | : 1690 $cm^{-1}$ |
| νNH | : 3360 $cm^{-1}$ |
| $\nu CH_2, CH_3$ | : 2990, 2940, 2880 $cm^{-1}$ |
| νC= C,C = N | : 1610, 1600, 1560 $cm^{-1}$ |
| δNH | : 1520 $cm^{-1}$ |

**Exemples 149 à 155 :**

En suivant les opérations décrites dans l'exemple 148, mais en remplaçant au stade B de l'exemple 1 la N-(4,6-diméthylpyridin-2-yl) isobutylamine par l'amine appropriée, on obtient successivement les produits des exemples suivants :

**Exemple 149 :**

**N-isobutyl N-(4,6-diméthylpyridin-2-yl) 2-(6-acétoxy-3,4-dihydro-2,5,7,8-tétraméthyl-2H[1]-benzopyran-2-yl) acétamide**

**Exemple 150 :**

**N-(2,4,6-triméthylphényl) 2-(6-acétoxy-3,4-dihydro-2,5,7,8-tétraméthyl-2H[1]-benzopyran-2-yl) acétamide**

**Exemple 151 :**

**N-(3,4,5-triméthoxyphényl) (6-acétoxy-3,4-dihydro-2,5,7,8-tétraméthyl-2H[1]-benzopyran-2-yl) acétamide**

**Exemple 152 :**

**N-hexyl N-(4,6-diméthylpyridin-2-yl) 2-(6-acétoxy-3,4-dihydro-2,5,7,8-tétraméthyl-2H[1]-benzopyran-2-yl) acétamide**

**Exemple 153 :**

**N-phényl N-(butèn-3 yl) 2-(6-acétoxy-3,4-dihydro-2,5,7,8-tétraméthyl-2H[1]-benzopyran-2-yl) acétamide**

**Exemple 154 :**

**N-isobutyl N-(4-méthylquinoléin-2-yl) 2-(6-acétoxy-3,4-dihydro-2,5,7,8-tétraméthyl-2H[1]-benzopyran-2-yl) acétamide**

**Exemple 155 :**

**N-allyl N-phényl 2-(6-acétoxy-3,4-dihydro-2,5,7,8-tétraméthyl-2H[1]-benzopyran-2-yl) acétamide**

**Exemples 156 à 163 :**

En opérant comme dans l'exemple 2, mais en remplaçant le composé de l'exemple 1 par le composé 148, 149, 150, 151, 152, 153, 154, puis 155, on obtient successivement les produits des exemples suivants :

**Exemple 156 :**

**N-(4-méthylquinoléin-2-yl) 2-(6-hydroxy-3,4-dihydro-2,5,7,8-tétraméthyl-2H[1]-benzopyran-2-yl) acétamide**

**Exemple 157 :**

**N-isobutyl N-(4,6-diméthylpyridin-2-yl) 2-(6-hydroxy-3,4-dihydro-2,5,7,8-tétraméthyl-2H[1]-benzopyran-2-yl) acétamide**

**Exemple 158 :**

**N-(2,4,6-triméthylphényl) 2-(6-hydroxy-3,4-dihydro-2,5,7,8-tétraméthyl-2H[1]-benzopyran-2-yl) acétamide**

**Exemple 159 :**

**N-(3,4,5-triméthoxyphényl) 2-(6-hydroxy-3,4-dihydro-2,5,7,8-tétraméthyl-2H[1]-benzopyran-2-yl) acétamide**

**Exemple 160 :**

**N-hexyl N-(4,6-diméthypyridin-2-yl) 2-(6-hydroxy-3,4-dihydro-2,5,7,8-tétraméthyl-2H[1]-benzopyran-2-yl) acétamide**

**Exemple 161 :**

**N-phényl N-(butèn-3 yl) 2-(6-hydroxy-3,4-dihydro-2,5,7,8-tétraméthyl-2H[1]-benzopyran-2-yl) acétamide**

**Exemple 162 :**

**N-isobutyl N-(4-méthylquinoléin-2-yl) 2-(6-hydroxy-3,4-dihydro-2,5,7,8-tétraméthyl-2H[1]-benzopyran-2-yl) acétamide**

**Exemple 163 :**

**N-allyl N-phényl 2-(6-hydroxy-3,4-dihydro-2,5,7,8-tétraméthyl-2H[1]-benzopyran-2-yl) acétamide**

**Exemple 164 :**

**N-(4,6-diméthylpyridin-2-yl) 2-(6-méthoxyméthoxy-3,4-dihydro-2,5,7,8-tétraméthyl-2H[1]-benzopyran-2-yl) acétamide**

En suivant les opérations décrites dans l'exemple 106, mais en remplaçant au stade A de l'exemple 106 l'acide (6-hydroxy-3,4-dihydro-2 ,5,7,8-tétraméthyl-2H[1]-benzopyran-2-yl) carboxylique par l'acide (6-hydroxy -3,4-dihydro-2,5,7,8-tétraméthyl-2H[1]-benzopyran-2-yl) acétique, on obtient le produit du titre.

**Exemples 165 à 168 :**

De même, en remplaçant au stade A de l'exemple 106 l'acide (6-hydroxy -3,4-dihydro-2,5,7,8-tétraméthyl-2H[1]-benzopyran-2-yl) carboxylique par l'acide (6-hydroxy-3,4-dihydro-2,5,7,8-tétraméthyl-2H[1]-benzopyran-2-yl) acétique, et en suivant les instructions des exemples 107 à 110, on obtient successivement les produits des exemples suivants :

**Exemple 165 :**

**N-(4,6-diméthylpyridin-2-yl) 2-(6-éthoxycarbonylméthoxy-3,4-dihydro-2,5,7,8-tétraméthyl-2H[1]-benzopyran-2-yl) acétamide**

**Exemple 166 :**

**N-(4,6-diméthylpyridin-2-yl) 2-(6-carboxyméthoxy-3,4-dihydro-2,5,7,8-tétraméthyl-2H[1]-benzopyran-2-yl) acétamide**

**Exemple 167 :**

**N-(4,6-diméthylpyridin-2-yl) 2-(6-éthoxyéthoxy-3,4-dihydro-2,5,7,8-tétraméthyl-2H[1]-benzopyran-2-yl) acétamide**

**Exemple 168 :**

**N-(4,6-diméthylpyridin-2-yl) 2-(6-éthoxycarbonyloxy-3,4-dihydro-2,5,7,8-tétraméthyl-2H[1]-benzopyran-2-yl) acétamide**

**Exemples 169 à 181 :**

En procédant comme dans l'exemple 121, mais en remplaçant successivement le composé de l'exemple 24 par les composés des exemples 156 à 168, on obtient respectivement les composés des exemples suivants :

**Exemple 169 :**

**N-(4-méthylquinoléin-2-yl) 2-(6-hydroxy-3,4-dihydro-2,5,7,8-tétraméthyl-2H[1]-benzopyran-2-yl) éthylamine**

**Exemple 170 :**

**N-isobutyl N-(4,6-diméthylpyridin-2-yl) 2-(6-hydroxy-3,4-dihydro-2,5,7,8-tétraméthyl-2H[1]-benzopyran-2-yl) éthylamine**

**Exemple 171 :**

**N-(2,4,6-triméthylphényl) 2-(6-hydroxy-3,4-dihydro-2,5,7,8-tétraméthyl-2H[1]-benzopyran-2-yl) éthylamine**

**Exemple 172 :**

**N-(3,4,5-triméthoxyphényl) 2-(6-hydroxy-3,4-dihydro-2,5,7,8-tétraméthyl-2H[1]-benzopyran-2-yl) éthylamine**

**Exemple 173 :**

**N-hexyl N-(4,6-diméthylpyridin-2-yl) 2-(6-hydroxy-3,4-dihydro-2,5,7,8-tétraméthyl-2H[1]-benzopyran-2-yl) éthylamine**

**Exemple 174 :**

**N-phényl N-(butèn-3 yl) 2-(6-hydroxy-3,4-dihydro-2,5,7,8-tétraméthyl-2H[1]-benzopyran-2-yl) éthylamine**

**Exemple 175 :**

**N-isobutyl N-(4-méthylquinoléin-2-yl) 2-(6-hydroxy-3,4-dihydro-2,5,7,8-tétraméthyl-2H[1]-benzopyran-2-yl) éthylamine**

**Exemple 176 :**

**N-allyl N-phényl 2-(6-hydroxy-3,4-dihydro-2,5,7,8-tétraméthyl-2H[1]-benzopyran-2-yl) éthylamine**

**Exemple 177 :**

**N-(4,6-diméthylpyridin-2-yl) 2-(6-méthoxyméthoxy-3, 4-dihydro-2,5,7,8-tétraméthyl-2H[1]-benzopyran-**

**2-yl) éthylamine**

**Exemple 178 :**

**N-(4,6-diméthylpyridin-2-yl) 2-(6-éthoxycarbonylméthoxy-3,4-dihydro-2,5,7,8-tétraméthyl-2H[1]-benzopyran-2-yl) éthylamine**

**Exemple 179 :**

**N-(4,6-diméthylpyridin-2-yl) 2-(6-carboxyméthoxy-3,4-dihydro-2,5,7,8-tétraméthyl-2H[1]-benzopyran-2-yl) éthylamine**

**Exemple 180 :**

**N-(4,6-diméthylpyridin-2-yl) 2-(6-éthoxyéthoxy-3,4-dihydro-2,5,7,8-tétraméthyl-2H[1]-benzopyran-2-yl) éthylamine**

**Exemple 181 :**

**N-(4,6-diméthylpyridin-2-yl) 2-(6-éthoxycarbonyloxy-3,4-dihydro-2,5,7,8-tétraméthyl-2H[1]-benzopyran-2-yl) éthylamine**

**Exemple 182 :**

**N-(4-méthylquinoléin-2-yl) (6-acétoxy-3,4-dihydro-5,7,8-triméthyl-2H[1]-benzopyran-2-yl) carboxamide**

En procédant comme dans l'exemple 1, mais en remplaçant au stade A de l'exemple 1 l'acide (6-hydroxy-3,4-dihydro-2,5,7,8-tétraméthyl-2H[1]-benzopyran-2yl) carboxylique par l'acide (6-hydroxy-3,4-dihydro-5,7,8-triméthyl-2H[1]-benzopyran-2-yl) carboxylique et en remplaçant au stade B de l'exemple 1 la N-(4,6-diméthylpyridin-2-yl) isobutylamine par la 2-amino-4-méthylquinoléine, ainsi qu'en remplaçant au même stade B de l'exemple 1 l'étape de chauffage au reflux, après le mélange de la solution du chlorure d'acide et de l'amine, par une agitation de 24 heures à la température de 20°C, on obtient le produit du titre.

**Exemples 183 à 185 :**

En suivant les opérations décrites dans l'exemple 182, mais en remplaçant au stade B de l'exemple 1 la N-(4,6-diméthylpyridin-2-yl) isobutylamine par l'amine appropriée on obtient successivement les produits des exemples suivants :

**Exemple 183 :**

**N-isobutyl N-(4-méthylquinoléin-2-yl) (6-acétoxy-3,4-dihydro-5,7,8-triméthyl-2H[1]-benzopyran-2-yl) carboxamide**

**Exemple 184 :**

**N-phényl N-(4,6-diméthylpyridin-2-yl) (6-acétoxy-3,4-dihydro-5,7,8-triméthyl-2H[1]-benzopyran-2-yl) carboxamide**

**Exemple 185 :**

**N-phényl N-(butèn-3 yl) (6-acétoxy-3,4-dihydro-5,7,8-triméthyl -2H[1]-benzopyran-2-yl) carboxamide**

**Exemples 186 à 189 :**

En opérant comme dans l'exemple 2, mais en remplaçant le composé de l'exemple 1 par le composé de l'exemple 182, 183, 184 puis 185, on obtient successivement les produits des exemples suivants :

**Exemple 186 :**

**N-(4-méthylquinoléin-2-yl) (6-hydroxy-3,4-dihydro-5,7,8-triméthyl-2H[1]-benzopyran-2-yl) carboxamide**

**Exemple 187 :**

**N-isobutyl N-(4-méthylquinoléin-2-yl) (6-hydroxy-3,4-dihydro-5,7,8-triméthyl-2H[1]-benzopyran-2-yl) carboxamide**

**Exemple 188 :**

**N-phényl N-(4,6-diméthylpyridin-2-yl) (6-hydroxy-5,7,8-triméthyl-2H[1]-benzopyran-2-yl) carboxamide**

**Exemple 189 :**

**N-phényl N-(butèn-3 yl) (6-hydroxy-3,4-dihydro-5,7,8-triméthyl-2H[1]-benzopyran-2-yl) carboxamide**

**Exemple 190 :**

**N-phényl (6-hydroxy-3,4-dihydro-2,5,7,8-tétraméthyl-2H[1]-benzopyran-2-yl)méthyl)amine**

En procédant comme dans l'exemple 121, mais en remplaçant le composé de l'exemple 24 par le composé de l'exemple 25, on obtient le produit du titre.

- Point de fusion (maléate) : 135°C

**Exemple 191 :**

**N-(2,6-diméthylphényl) (6-acétoxy-3,4-dihydro-2,5,7,8-tétraméthyl-2H[1]-benzopyran-2-yl) carboxamide**

En remplaçant dans le stade B de l'exemple 1 la N-(4,6-diméthylpyridin-2-yl)isobutylamine par la 2,5-diméthylaniline, on obtient le produit du titre.

**Exemple 192 :**

**N-(2,6-diméthylphényl) (6-hydroxy-3,4-dihydro-2,5,7,8-tétraméthyl-2H[1]-benzopyran-2-yl) carboxamide**

En opérant comme dans l'exemple 2, mais en remplaçant le composé de l'exemple 1 par le composé de l'exemple 191, on obtient le produit du titre.

- Rendement : 61 %
- Point de fusion : 170°C
- Caractéristiques spectrales en infra rouge :

νOH, NH : 3400, 3360 $cm^{-1}$
ν$CH_3$ : 2970, 2910, 2850 $cm^{-1}$
νC=O : 1670 $cm^{-1}$
νC=C : 1605, 1585, 1590 $cm^{-1}$

**Exemple 193 :**

**N-phényl (6-acétoxy-3,4-dihydro-2-méthyl-7-tert. butyl-2H[1]-benzopyran-2-yl) carboxamide**

En procédant comme dans l'exemple 1, mais en remplaçant au stade A de l'exemple 1 l'acide (6-hydroxy-3,4-dihydro-2,5,7,8-tétraméthyl-2H[1]benzopyran-2-yl) carboxylique par l'acide (6-hydroxy-3,4-dihydro-2-méthyl-7-tert.butyl-2H[1]-benzopyran-2-yl) carboxylique et en remplaçant au stade B de l'exemple 1 la N-(4,6-diméthylpyridin-2-yl)isobutylamine par l'aniline, on obtient le produit du titre.

**Exemple 194 :**

**N-phényl (6-hydroxy-3,4-dihydro-2-méthyl-7-tert. butyl-2H[1]-benzopyran-2-yl) carboxamide**

En opérant comme dans l'exemple 2, mais en remplaçant le composé de l'exemple 1 par le composé de

l'exemple 193, on obtient le produit du titre.

- Point de fusion : 129-131°C
- Caractéristiques spectrales en infra rouge :

νNH,OH : 3380, 3330 $cm^{-1}$
$\nu CH_3$ : 2940, 2860 $cm^{-1}$
νC = O : 1655 $cm^{-1}$
νC = C : 1615, 1590 $cm^{-1}$

## ETUDE PHARMACOLOGIQUE DES COMPOSES DE L'INVENTION

**(Les composés sont comparés avec le composé le plus proche de l'art antérieur qui est l'exemple 102 de la demande WO 88/08424)**

**Exemple 195 :**

**ETUDE DE L'ACTIVITE ANTIPEROXYDANTE**

L'action des composés de l'invention, susceptibles de piéger les radicaux ·OH a été étudiée d'une part, sur la peroxydation spontanée des lipides et d'autre part, sur la peroxydation induite par le système $Fe^{2+}$ - ascorbate (10 μM - 250 μM), et ce sur des homogénats de cerveaux de rats.

Lors de la mesure de la peroxydation lipidique spontanée, les homogénats de cerveau de rats sont placés en présence ou en absence des composés à tester durant 60 minutes à 37°C. La réaction est arrêtée à 0°C et le dosage du malondialdéhyde est effectué à l'aide d'acide thiobarbiturique par la méthode de YAGI, K (1976) Biochem. Med, 15 212 -216. La peroxydation lipidique est déterminée par les substances réagissant avec l'acide thiobarbiturique exprimées en nanomoles de malondialdéhyde.

Lors de la mesure de la peroxydation lipidique induite, la méthodologie est identique à la précédente à l'exception de l'addition à l'homogénat du système inducteur de radicaux : $Fe^{2+}$ - ascorbate. Les substances de référence sont le probucol et la vitamine E.

Les concentrations des composés testés inhibant de 50 % la peroxydation du substrat sont calculées.

Il est apparu que certains composés de l'invention possèdent une activité antiperoxydante particulièrement intense puisque supérieure d'un facteur 100 à celle du composé le plus proche de l'art antérieur. Ce résultat très intéressant se produit que la peroxydation soit spontanée ou induite par un système chimique.

**Exemple 196 :**

**ETUDE DU POUVOIR PROTECTEUR DE L'OXYDATION DES LDL**

La capacité des composés de l'invention à diminuer les proportions de LDL oxydées a été mesurée de la façon suivante. On réalise une incubation de 24 heures regroupant des LDL natives, un système $Cu^{2+}$ générateur de radicaux libres et les composés à tester.

Les résultats sont obtenus après analyse du milieu par une technique chromatographique haute performance : la FPLC (Fast Protein Liquid Chromatography). Le pouvoir protecteur du composé testé est déterminé après comparaison du chromatogramme obtenu avec celui du témoin positif de référence : le probucol.

Il apparaît clairement que les composés de l'invention ont un pouvoir protecteur très important et significativement supérieur à celui du composé le plus proche de l'art antérieur.

**Exemple 197 :**

**ETUDE DE L'ACTIVITE INHIBITRICE DES COMPOSES SUR LA BIOSYNTHESE DE PROSTANOIDES**

La recherche de l'activité inhibitrice des composés sur la biosynthèse des prostanoïdes a été réalisée sur des plaquettes humaines préalablement activées par la thrombine et mises en présence des produits à tester.

La production de thromboxane $B_2$, qui est un prostanoïde majeur élaboré par les plaquettes, est déterminée par dosage radioimmunologique (RIA).

Les composés de l'invention inhibent la production du thromboxane $B_2$ de façon très importante alors que le composé le plus proche de l'art antérieur n'a aucun effet sur cette production.

**Exemple 198 :**

**COMPOSITION PHARMACEUTIQUE : COMPRIMES**

Comprimés dosés à 50 mg de N-isobutyl N-(4,6-diméthylpyridin-2-yl) (6-hydroxy-3,4-dihydro-2,5,7,8-tétraméthyl-2H[1]-benzopyran-2-yl) carboxamide
Formule de préparation pour 1 000 comprimés :

| | |
|---|---|
| N-isobutyl N-(4,6-diméthylpyridin-2-yl) (6-hydroxy-3,4-dihydro-2,5,7,8-tétraméthyl-2H[1]-benzopyran-2-yl) carboxamide | 50g |
| Amidon de blé | 15g |
| Amidon de maïs | 15g |
| Lactose | 65g |
| Stéarate de Magnesium | 2g |
| Silice | 1g |
| Hydroxypropylcellulose | 2g |

## Revendications

**1)** Composés de formule générale (I) :

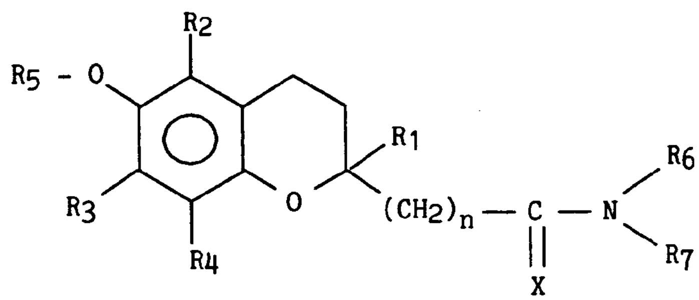

(I)

dans laquelle :
n représente un nombre entier égal à 0 ou 1,
X représente un atome d'oxygène ou 2 atomes d'hydrogène,

$R_1$, $R_2$, $R_3$, $R_4$, identiques ou différents, représentent chacun indépendamment l'un de l'autre un atome d'hydrogène ou un radical alkyl inférieur $R_a$-, où $R_a$ représente un groupement alkyle linéaire où ramifié de 1 à 8 atomes de carbone,

$R_5$ représente :
- un atome d'hydrogène,
- un groupement alkyle inférieur $R_a$-,
- un groupement acyle inférieur $R_a$-CO-,
- un groupement alcoxyalkyle de forme $R_a$-O-$R_b$-,
- un groupement alcoxycarbonyle de forme $R_a$-O-CO-,
- un groupement alcoxycarbonylalkyle de forme $R_a$-O-CO-$R_b$-,
- un groupement carboxylalkyle de forme HOOC-$R_a$-,

où $R_a$ et $R_b$, identiques ou différents, représentent chacun indépendamment l'un de l'autre, un radical alkyle linéaire ou ramifié comportant de 1 à 8 atomes de carbone,

$R_6$ et $R_7$
- soit :

forment ensemble avec l'atome d'azote qui les porte un groupement E ou un groupement E substitué avec E représentant un groupement hétérocyclique spirannique de 8 à 12 chaînons incluant dans son squelette carboné de 1 à 4 hétéroatomes choisis parmi azote, soufre, et oxygène,
- soit :

identiques ou différents, représentent chacun indépendamment l'un de l'autre :
- un atome d'hydrogène,

- un groupement alkyle inférieur Ra- ou un groupement alkyle inférieur Ra- substitué,
- un groupement alcényle inférieur ou un groupement alcényle inférieur substitué, où le groupement alcényle représente un hydrocarbure insaturé, linéaire ou ramifié, comprenant 2 à 8 atomes de carbone,
- un groupement A-$(CH_2)_m$- ou un groupement A-$(CH_2)_m$- substitué, où m est un nombre entier égal à 0,1, ou 2 et A représente un groupement cycloalkyle comportant p atomes de carbone avec p étant un nombre entier de 3 à 7 étant entendu que si p est 3 ou 4 alors m peut être 0, 1, ou 2 et si p est 5,6, ou 7 alors m ne peut être que 1 ou 2,
- un groupement alcoxyalkyle de forme $R_a$-O-$R_b$- ou un groupement alcoxyalkyle de forme $R_a$-O-$R_b$- substitué, où $R_a$ et $R_b$, identiques ou différents, représentent un radical alkyle inférieur, linéaire ou ramifié de, 1 à 8 atomes de carbone,
- un groupement alcoxycarbonylalkyle de forme $R_a$-O-CO-$R_b$- ou un groupement alcoxycarbonylalkyle de forme $R_a$-O-CO-$R_b$- substitué, avec $R_a$ et $R_b$ tels que définis ci-dessus,
- un groupement B-$(CH_2)_q$- ou un groupement B-$(CH_2)_q$- substitué, où q est un nombre entier égal à 0, 1, 2, ou 3 et B représente un radical naphtalène, 1,3-dioxane , pyrane, ou benzopyrane,
- un groupement E-$(CH_2)_q$- ou un groupement E-$(CH_2)_q$- substitué, avec q et E tels que définis précédemment,
- un groupement phényl-$(CH_2)_q$- ou un groupement phényl-$(CH_2)_q$- substitué, avec q tel que défini ci-dessus,
- un groupement hétéroaryl-$(CH_2)_q$- ou un groupement hétéroaryl-$(CH_2)_q$-substitué, avec q tel que défini ci-dessus et où l'hétéroaryle est choisi parmi :
furane, quinoléine, isoquinoléine, pyridine, thiophène, thiazole, isothiazole, oxazole, isoxazole, naphtyridine, benzofurane, β-carboline , ou γ-carboline,
- un des radicaux $D_1$ à $D_9$ suivants ou un des radicaux $D_1$ à $D_9$ suivants substitué :

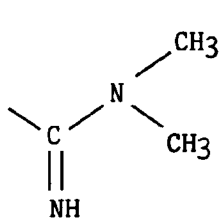 , 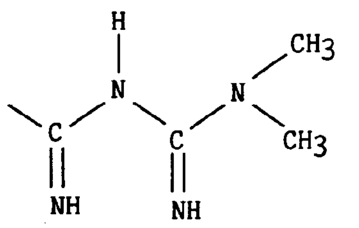 , 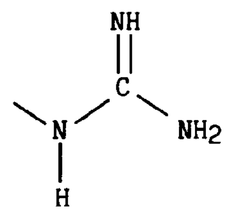 ,

$D_1$ $D_2$ $D_3$

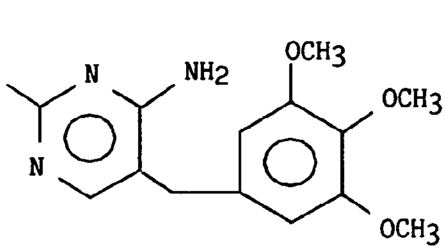 , 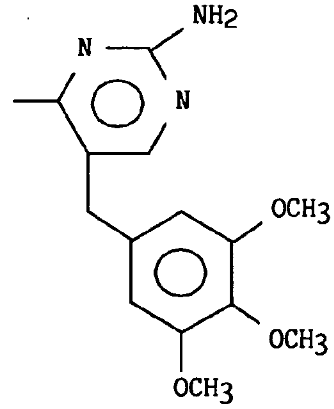 ,

$D_4$ $D_5$

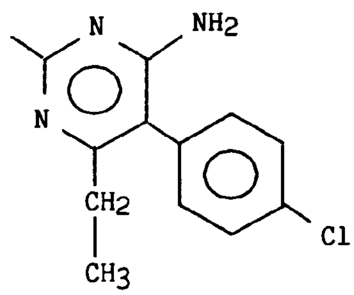

D6

,

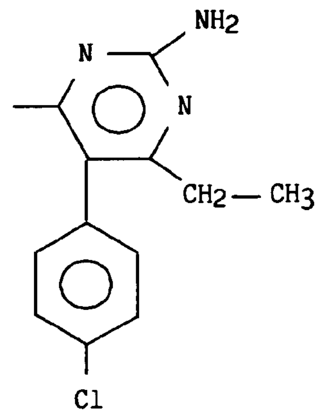

D7

,

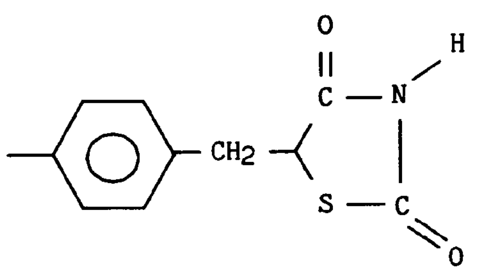

D8

,

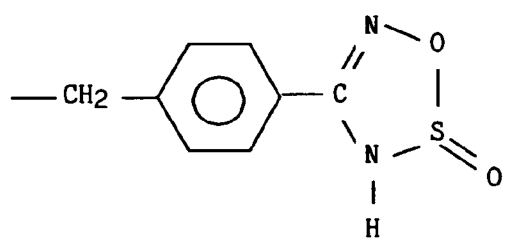

D9

,

étant entendu que

lorsque l'un des substituants $R_6$ ou $R_7$ représente un atome d'hydrogène, un groupement alkyle inférieur comportant au maximum 3 atomes de carbone, un groupement phényl-$(CH_2)_{q'}$- non substitué, un groupement phényl-$(CH_2)_{q'}$- substitué par 1 ou 2 radicaux, un groupement pyridinyl-$(CH_2)_q$- non substitué, ou un groupement pyridinyl-$(CH_2)_q$- substitué par 1 ou 2 radicaux, avec q tel que défini précédemment et q' étant un nombre entier égal à 1, 2, ou 3,

alors l'une au moins des 2 conditions suivantes est vérifiée :

- ou bien l'autre substituant, $R_6$ ou $R_7$ selon le cas, ne représente pas : un atome d'hydrogène, un groupement alkyle inférieur comportant au maximum 3 atomes de carbone, un groupement phényl-$(CH_2)_{q'}$- non substitué, un groupement phényl-$(CH_2)_{q'}$- substitué par 1 ou 2 radicaux, un groupement pyridinyl-$(CH_2)_q$- non substitué, un groupement pyridinyl-$(CH_2)_q$- substitué par 1 ou 2 radicaux, avec q et q' tels que définis précédemment,
- ou bien le substituant $R_5$ ne représente pas un atome d'hydrogène, un groupement alkyle inférieur comportant au maximum 4 atomes de carbone, ou un groupement acyle inférieur $R'_5$-CO- avec $R'_5$ représentant un radical alkyle inférieur comportant au maximum 4 atomes de carbone,

étant entendu que

lors de cette description de la formule générale (I), le terme "substitué" affectant les groupements tels que définis précédemment : alkyle inférieur Ra-, alcényle inférieur, A-$(CH_2)_m$-, alcoxyalkyle $R_a$-O-$R_b$-, alcoxycarbonylalkyle $R_a$-O-CO-$R_b$-, B-$(CH_2)_q$-, E-$(CH_2)_q$-, phényl-$(CH_2)_q$-, phényl-$(CH_2)_{q'}$-, hétéroaryl-$(CH_2)_q$, $D_1$ à $D_9$, signifie, lorsque cela n'est pas précisé, que ces groupements peuvent être substitués par un ou plusieurs radicaux, identiques ou différents, et représentant chacun indépendamment l'un de l'autre :

un groupement alkyle inférieur $R_c$,

alcoxy inférieur $R_c$-O-,
acyle inférieur $R_c$-CO-,
trifluorométhyle,
carboxyle,
hydroxyle,
amino,
amino substitué par 1 ou 2 groupement alkyles inférieurs $R_c$-,
nitro,
oxo,
phényle,
alkylthio inférieur $R_c$-S- ,
thiol,
ou un atome d'halogène,
où $R_c$ représente un groupement alkyle linéaire ou ramifié de 1 à 6 atomes de carbone,
leurs isomères optiques,

ainsi que, le cas échéant, leurs sels d'addition à un acide ou à une base pharmaceutiquement acceptable.

**2)** Composés selon la revendication 1 pour lesquels $R_5$ représente un atome d'hydrogène, leurs isomères optiques et leurs sels d'addition à une base ou à un acide pharmaceutiquement acceptable.

**3)** Composés selon la revendication 1 pour lesquels X représente un atome d'oxygène, leurs isomères optiques et leurs éventuels sels d'addition à une base ou à un acide pharmaceutiquement acceptable.

**4)** Composés selon la revendication 1 pour lesquels $R_5$ représente un atome d'hydrogène et X représente un atome d'oxygène, leurs isomères optiques et leurs éventuels sels d'addition à une base ou à un acide pharmaceutiquement acceptable.

**5)** Composés selon la revendication 1 pour lesquels au moins 1 des 2 substituants $R_6$ ou $R_7$ représente un groupement pyridinyle ou un groupement pyridinyle substitué, leurs isomères optiques et leurs éventuels sels d'addition à une base ou à un acide, pharmaceutiquement acceptable.

**6)** Composés selon la revendication 1 pour lesquels au moins 1 des 2 substituants $R_6$ ou $R_7$ représente un groupement quinoléinyle ou un groupement quinoléinyle substitué, leurs isomères optiques et leurs éventuels sels d'addition à une base ou à un acide pharmaceutiquement acceptable.

**7)** Composés selon la revendication 1 pour lesquels au moins 1 des 2 substituants $R_6$ ou $R_7$ représente un groupement phényle ou un groupement phényle substitué, leurs isomères optiques et leurs éventuels sels d'addition à une base ou à un acide pharmaceutiquement acceptable.

**8)** Le N-isobutyl N-(4,6-diméthylpyridin-2-yl) (6-hydroxy-3,4-dihydro-2,5,7,8-tétraméthyl-2H[1]-benzopyran-2-yl) carboxamide, ses isomères optiques et ses sels d'addition à une base pharmaceutiquement acceptable.

**9)** le N-(3,4,5-triméthoxyphényl) (6-hydroxy-3,4-dihydro-2,5,7,8-tétraméthyl-2H[1]-benzopyran-2-yl) carboxamide, ses isomères optiques et ses sels d'addition à une base pharmaceutiquement acceptable.

**10)** le N-(4-méthylquinoléin-2-yl) (6-hydroxy-3,4-dihydro-2,5,7,8-tétraméthyl-2H[1]-benzopyran-2-yl) carboxamide, ses isomères optiques et ses sels d'addition à une base pharmaceutiquement acceptable.

**11)** le N-{4-[(2,4-dioxo-5-thiazolidinyl)méthyl]phényl} (6-hydroxy-3,4-dihydro-2,5,7,8-tétraméthyl-2H[1]-benzopyran-2-yl) carboxamide, ses isomères optiques et ses sels d'addition à une base pharmaceutiquement acceptable.

**12)** le N-(méthoxyéthyl) (6-hydroxy-3,4-dihydro-2,5,7,8-tétraméthyl-2H[1]-benzopyran-2-yl) carboxamide, ses isomères optiques et ses sels d'addition à une base pharmaceutiquement acceptable.

**13)** Procédé d'obtention des composés de formule (I) caractérisé en ce que l'on utilise comme matière première un composé de formule (II) :

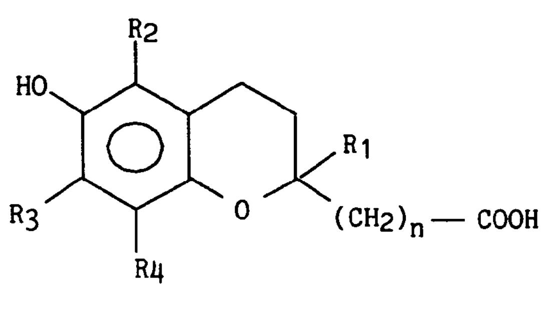

(II)

avec $R_1$, $R_2$, $R_3$, $R_4$, et n ayant la même définition que dans la formule générale (I),

que l'on peut éthérifier ou estérifier, en milieu basique anhydre, par un composé $R_{5''}$-Hal ou $R_{5''}$-O-$R_{5''}$, où Hal représente un atome d'halogène et où $R_{5''}$ représente un groupement alkyle inférieur $R_a$-, un groupement acyle inférieur $R_a$-CO-, un groupement alcoxyalkyle de forme $R_a$-O-$R_b$-, un groupement alcoxycarbonyle de forme $R_a$-O-CO-, un groupement alcoxycarbonylalkyle de forme $R_a$-O-CO-$R_b$-, un groupement carboxylalkyle de forme HOOC-$R_a$-, formules dans lesquelles $R_a$ et $R_b$, identiques ou différents, représentent chacun indépendamment l'un de l'autre un groupement alkyle inférieur, linéaire ou ramifié, comprenant de 1 à 8 atomes de carbone, pour obtenir un composé de formule (III) :

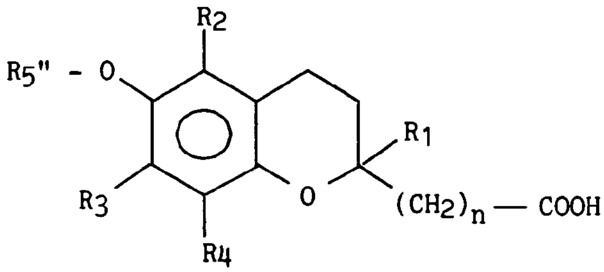

(III)

avec $R_1$, $R_2$, $R_3$, $R_4$, n, et $R_{5''}$ tels que définis précédemment, qui est transformé en son chlorure par action d'un agent d'halogénation, puis traité, dans un solvant approprié, en présence d'un agent alcalin, par une amine de formule (IV) :

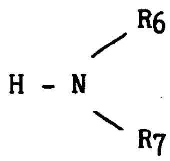

(IV)

avec $R_6$ et $R_7$ ayant la même signification que dans la formule (I) pour obtenir un composé de formule ($I_a$) :

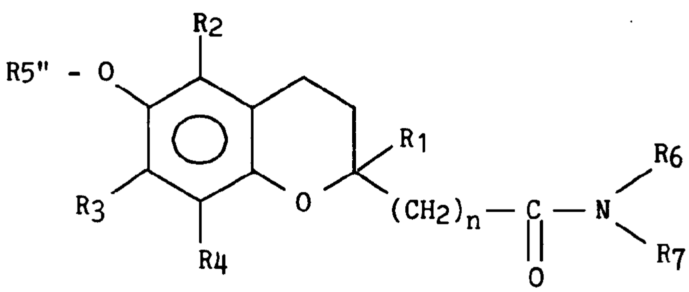

($I_a$)

avec $R_1$, $R_2$, $R_3$, $R_4$, $R_5$, $R_7$, n, et $R_{5''}$ tels que définis précédemment, cas particulier des composés de formule (I) pour lesquels $R_5$ représente un groupement $R_{5''}$ et X représente un atome d'oxygène,

que l'on peut ensuite :

- soit réduire par action d'un hydrure mixte de métal alcalin en un composé de formule ($I_b$) :

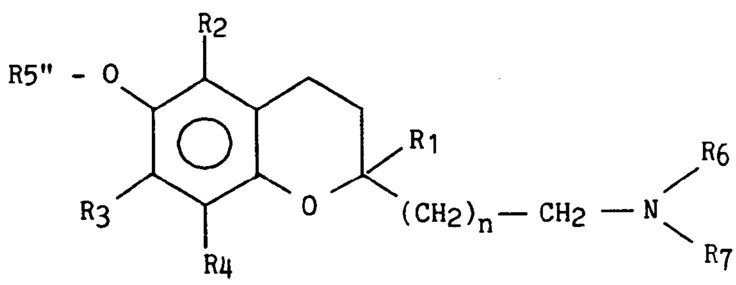

($I_b$)

avec $R_1$, $R_2$, $R_3$, $R_4$, $R_5$, $R_7$, n, et $R_{5''}$ tels que définis précédemment, cas particulier des composés de for-

mule (I) pour lesquels $R_5$ représente un groupement $R_{5''}$ et X représente deux atomes d'hydrogène,
- soit saponifier par action d'un hydroxyde de métal alcalin ou alcalino-terreux en un composé de formule ($I_c$) :

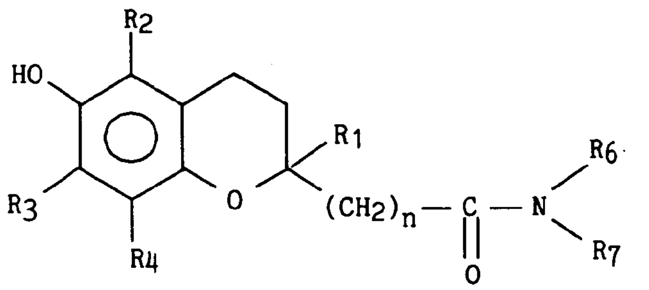

($I_c$)

avec $R_1$, $R_2$, $R_3$, $R_4$, $R_6$, $R_7$, et n tels que définis précédemment,

cas particulier des composés de formule (I) pour lesquels $R_5$ représente un atome d'hydrogène et X représente un atome d'oxygène, composé ($I_c$) qui peut être ensuite :

- ou bien réduit par action d'un hydrure mixte de métal alcalin en un dérivé de formule ($I_d$) :

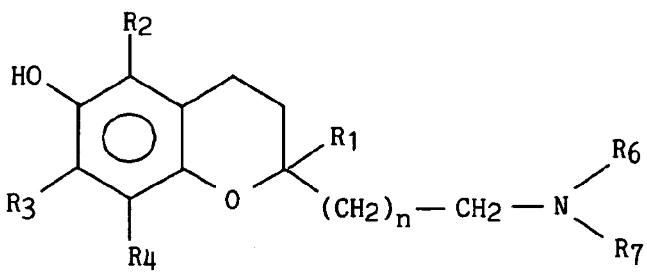

($I_d$)

avec $R_1$, $R_2$, $R_3$, $R_4$, $R_6$, $R_7$, et n tels que définis précédemment, cas particulier des composés de formule (I) pour lesquels $R_5$ représente un atome d'hydrogène et X représente 2 atomes d'hydrogène,
- ou bien éthérifié ou estérifié, afin d'obtenir, si on le désire, un groupement $R_5$ différent de celui présent dans la formule (Ib), par action d'un dérivé de formule $R_{5'''}$-O- $R_{5'''}$ ou $R_{5'''}$-Hal, où Hal représente un atome d'halogène et $R_{5'''}$ représente un groupement acyle inférieur $R_a$-CO-, un groupement alcoxyalkyle de forme $R_a$-O-$R_b$-, un groupement alcoxycarbonylalkyle de forme $R_a$-O-CO-$R_b$-, ou un groupement carboxylalkyle de forme HOOC-$R_a$-, avec $R_a$ et $R_b$ tels que définis précédemment, pour obtenir un composé de formule ($I_e$) :

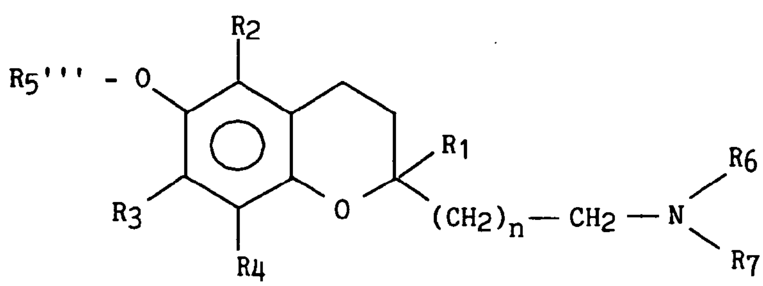

($I_e$)

avec $R_1$, $R_2$, $R_3$, $R_4$, $R_5$, $R_7$, n, et $R_{5'''}$ tels que définis précédemment, cas particulier des composés de formule (I) pour lesquels $R_5$ représente un groupement $R_{5'''}$ et X représente 2 atomes d'hydrogène,

les composés de formule (I

$_a$), ($I_b$), ($I_c$), ($I_d$), et ($I_e$) formant l'ensemble des composés de formule (I) qui peuvent être, si on le désire, purifiés et/ou séparés en leurs isomères optiques,

ces composés de formule (I) pouvant également être, le cas échéant, transformés en leurs sels d'addition à une base ou à un acide pharmaceutiquement acceptable.

**14)** Composition pharmaceutique contenant comme principe actif au moins un composé selon l'une des

revendications 1 à 12, seul ou en combinaison avec un ou plusieurs excipients inertes non toxiques.

**15)** Composition pharmaceutique selon la revendication 14 utilisable dans le traitement et la prévention des affections dues ou reliées à des phénomènes de peroxydation ou de dérèglement de la biosynthèse des prostanoïdes, c'est à dire les désordres ischémiques centraux ou périphériques, les maladies inflammatoires, l'arthrite rhumatoïde, les maladies métaboliques, l'athérome, l'artériosclérose, les maladies respiratoires, l'asthme, l'emphysème, les maladies d'origine immunologique, le lupus érythémateux, les réactions allergiques, certains cancers, le vieillissement cérébral ou cutané, ainsi que dans la prévention et le traitement des dommages dus aux traumatismes chirurgicaux tels que la reperfusion d'organes.

**Revendications pour l'Etat contractant suivant : GR**

**1)** Procédé de préparation des composés de formule générale (I) :

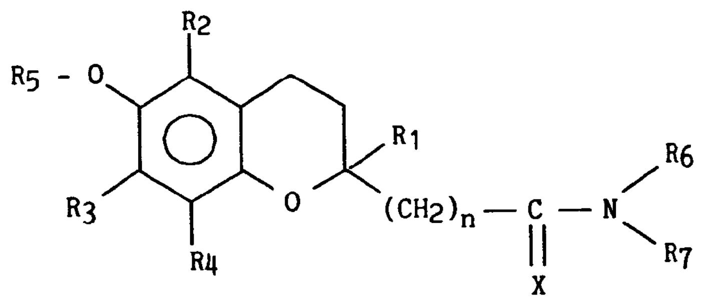

(I)

dans laquelle :
n représente un nombre entier égal à 0 ou 1,
X représente un atome d'oxygène ou 2 atomes d'hydrogène,

$R_1$, $R_2$, $R_3$, $R_4$, identiques ou différents, représentent chacun indépendamment l'un de l'autre un atome d'hydrogène ou un radical alkyl inférieur $R_a$-, où $R_a$ représente un groupement alkyle linéaire où ramifié de 1 à 8 atomes de carbone,

$R_5$ représente :
- un atome d'hydrogène,
- un groupement alkyle inférieur $R_a$-,
- un groupement acyle inférieur $R_a$-CO-,
- un groupement alcoxyalkyle de forme $R_a$-O-$R_b$-,
- un groupement alcoxycarbonyle de forme $R_a$-O-CO-,
- un groupement alcoxycarbonylalkyle de forme $R_a$-O-CO-$R_b$-,
- un groupement carboxylalkyle de forme HOOC-$R_a$-,

où $R_a$ et $R_b$, identiques ou différents, représentent chacun indépendamment l'un de l'autre, un radical alkyle linéaire ou ramifié comportant de 1 à 8 atomes de carbone,

$R_6$ et $R_7$

- soit :

forment ensemble avec l'atome d'azote qui les porte un groupement E ou un groupement E substitué avec E représentant un groupement hétérocyclique spirannique de 8 à 12 chaînons incluant dans son squelette carbone de 1 à 4 hétéroatomes choisis parmi azote, soufre, et oxygène,

- soit :

identiques ou différents, représentent chacun indépendamment l'un de l'autre :
- un atome d'hydrogène,
- un groupement alkyle inférieur Ra- ou un groupement alkyle inférieur Ra- substitué,
- un groupement alcényle inférieur ou un groupement alcényle inférieur substitué, où le groupement alcényle représente un hydrocarbure insaturé, linéaire ou ramifié, comprenant 2 à 8 atomes de carbone,
- un groupement A-($CH_2$)m- ou un groupement A-$(CH_2)_m$- substitué, où m est un nombre entier égal à 0,1, ou 2 et A représente un groupement cycloalkyle comportant p atomes de carbone avec p étant un nombre entier de 3 à 7 étant entendu que si p est 3 ou 4 alors m peut être 0, 1, ou 2 et si p est 5,6, ou 7 alors m ne peut être que 1 ou 2,
- un groupement alcoxyalkyle de forme $R_a$-O-$R_b$- ou un groupement alcoxyalkyle de forme $R_a$-O-$R_b$- substitué, où $R_a$ et $R_b$, identiques ou différents, représentent un radical alkyle inférieur, linéaire ou ramifié de, 1 à 8 atomes de carbone,

- un groupement alcoxycarbonylalkyle de forme $R_a$-O-CO-$R_b$- ou un groupement alcoxycarbonylalkyle de forme $R_a$-O-CO-$R_b$- substitue, avec $R_a$ et $R_b$ tels que définis ci-dessus,
- un groupement B-$(CH_2)_q$- ou un groupement B-$(CH_2)_q$- substitué, où q est un nombre entier égal à 0, 1, 2, ou 3 et B représente un radical naphtalène, 1,3-dioxane , pyrane, ou benzopyrane,
- un groupement E-$(CH_2)_q$- ou un groupement E-$(CH_2)_q$- substitué, avec q et E tels que définis précédemment,
- un groupement phényl-$(CH_2)_q$- ou un groupement phényl-$(CH_2)_q$- substitué, avec q tel que défini ci-dessus,
- un groupement hétéroaryl-$(CH_2)_q$- ou un groupement hétéroaryl-$(CH_2)_q$- substitué, avec q tel que défini ci-dessus et où l'hétéroaryle est choisi parmi :
furane, quinoléine, isoquinoléine, pyridine, thiophène, thiazole, isothiazole, oxazole, isoxazole, naphtyridine, benzofurane, β-carboline , ou γ-carboline,
- un des radicaux $D_1$ à $D_9$ suivants ou un des radicaux $D_1$ à $D_9$ suivants substitué :

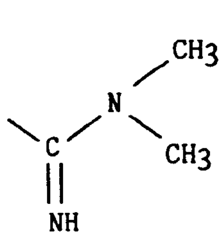
,
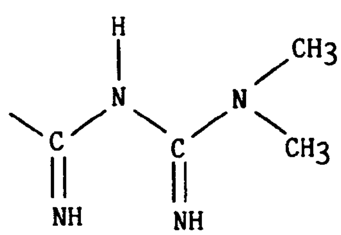
,
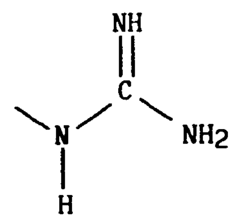
,

D1

D2

D3

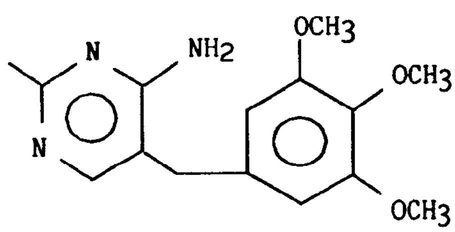
,
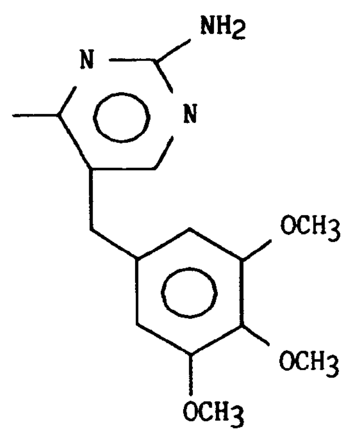
,

D4

D5

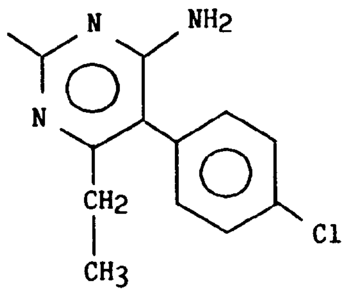

D6

,

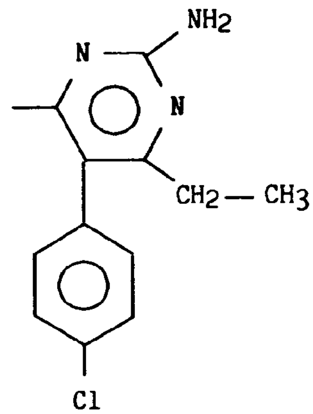

D7

,

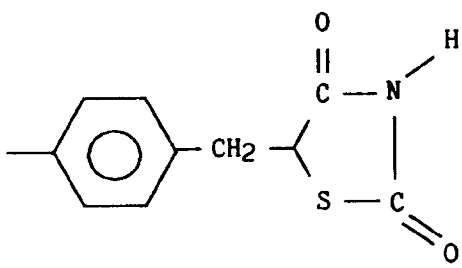

D8

,

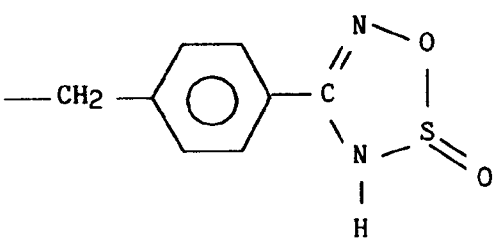

D9

,

étant entendu que

lorsque l'un des substituants $R_6$ ou $R_7$ représente un atome d'hydrogène, un groupement alkyle inférieur comportant au maximum 3 atomes de carbone, un groupement phényl-$(CH_2)_{q'}$- non substitué, un groupement phényl-$(CH_2)_{q'}$- substitué par 1 ou 2 radicaux, un groupement pyridinyl-$(CH_2)_q$- non substitué, ou un groupement pyridinyl-$(CH_2)_q$- substitué par 1 ou 2 radicaux, avec q tel que défini précédemment et q' étant un nombre entier égal à 1, 2, ou 3,

alors l'une au moins des 2 conditions suivantes est vérifiée :

- ou bien l'autre substituant, $R_6$ ou $R_7$ selon le cas, ne représente pas : un atome d'hydrogène, un groupement alkyle inférieur comportant au maximum 3 atomes de carbone, un groupement phényl-$(CH_2)_{q'}$- non substitué, un groupement phényl-$(CH_2)_{q'}$- substitue par 1 ou 2 radicaux, un groupement pyridinyl-$(CH_2)_q$- non substitué, un groupement pyridinyl-$(CH_2)_q$- substitué par 1 ou 2 radicaux, avec q et q' tels que définis précédemment,
- ou bien le substituant $R_5$ ne représente pas un atome d'hydrogène, un groupement alkyle inférieur comportant au maximum 4 atomes de carbone, ou un groupement acyle inférieur $R'_5$-CO- avec $R'_5$ représentant un radical alkyle inférieur comportant au maximum 4 atomes de carbone,

étant entendu que

lors de cette description de la formule générale (I), le terme "substitué" affectant les groupements tels que définis précédemment : alkyle inférieur Ra-, alcényle inférieur, A-$(CH_2)_m$-, alcoxyalkyle $R_a$-O-$R_b$-, alcoxycarbonylalkyle $R_a$-O-CO-$R_b$-, B-$(CH_2)_q$-, E-$(CH_2)_q$-, phényl-$(CH_2)_q$-, phényl-$(CH_2)_{q'}$-, hétéroaryl-$(CH_2)_q$, $D_1$ à $D_9$, signifie, lorsque cela n'est pas précisé, que ces groupements peuvent être substitues par un ou plusieurs radicaux, identiques ou différents, et représentant chacun indépendamment l'un de l'autre :

un groupement alkyle inférieur $R_c$,
alcoxy inférieur $R_c$-O-,
acyle inférieur $R_c$-CO-,
trifluorométhyle,
carboxyle,
hydroxyle,
amino,
amino substitué par 1 ou 2 groupement alkyles inférieurs $R_c$-,
nitro,
oxo,
phényle,
alkylthio inférieur $R_c$-S- ,
thiol,
ou un atome d'halogène,
où $R_c$ représente un groupement alkyle linéaire ou ramifié de 1 à 6 atomes de carbone,

de leurs isomères optiques,
ainsi que, le cas echéant, de leurs sels d'addition à un acide ou à une base pharmaceutiquement acceptable,

caractérisé en ce que :
on utilise comme matière première un composé de formule (II) :

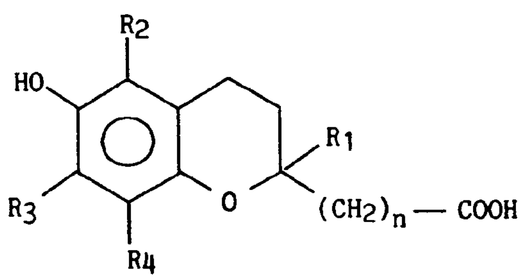

(II)

avec $R_1$, $R_2$, $R_3$, $R_4$, et n ayant la même définition que dans la formule générale (I),
que l'on peut éthérifier ou estérifier, en milieu basique anhydre, par un composé $R_{5''}$-Hal ou $R_{5''}$-O-$R_{5''}$, où Hal représente un atome d'halogène et où $R_{5''}$ représente un groupement alkyle inférieur $R_a$-, un groupement acyle inférieur $R_a$-CO-, un groupement alcoxyalkyle de forme $R_a$-O-$R_b$-, un groupement alcoxycarbonyle de forme $R_a$-O-CO-, un groupement alcoxycarbonylalkyle de forme $R_a$-O-CO-$R_b$-, un groupement carboxylalkyle de forme HOOC-$R_a$-, formules dans lesquelles $R_a$ et $R_b$, identiques ou différents, représentent chacun indépendamment l'un de l'autre un groupement alkyle inférieur, linéaire ou ramifié, comprenant de 1 à 8 atomes de carbone,
pour obtenir un composé de formule (III) :

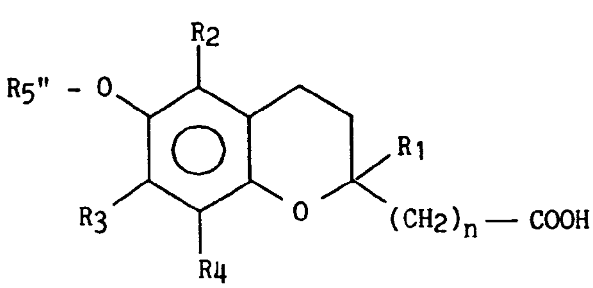

(III)

avec $R_1$, $R_2$, $R_3$, $R_4$, n, et $R_{5''}$ tels que définis précédemment, qui est transformé en son chlorure par action d'un agent d'halogénation, puis traité, dans un solvant approprié, en présence d'un agent alcalin, par une amine de formule (IV) :

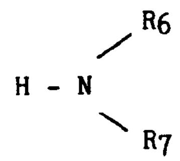

(IV)

avec $R_6$ et $R_7$ ayant la même signification que dans la formule (I) pour obtenir un compose de formule ($I_a$) :

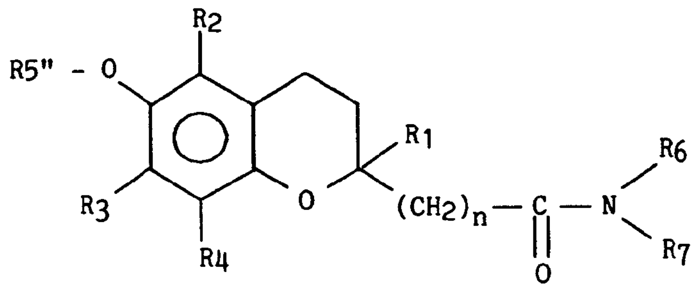

($I_a$)

avec $R_1$, $R_2$, $R_3$, $R_4$, $R_6$, $R_7$, n, et $R_{6''}$ tels que définis précédemment, cas particulier des composés de formule (I) pour lesquels $R_6$ représente un groupement $R_{6''}$ et X représente un atome d'oxygène,

que l'on peut ensuite :

- soit réduire par action d'un hydrure mixte de métal alcalin en un composé de formule ($I_b$) :

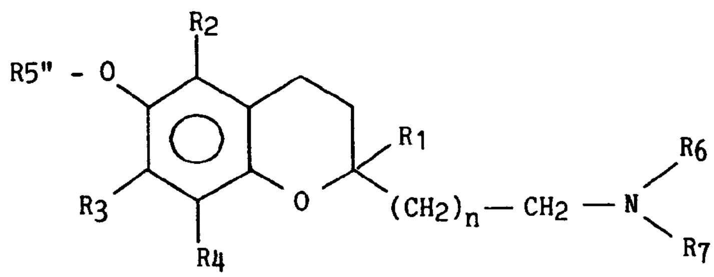

($I_b$)

avec $R_1$, $R_2$, $R_3$, $R_4$, $R_6$, $R_7$, n, et $R_{6''}$ tels que définis précédemment, cas particulier des composés de formule (I) pour lesquels $R_6$ représente un groupement $R_{6''}$ et X représente deux atomes d'hydrogène,

- soit saponifier par action d'un hydroxyde de métal alcalin ou alcalino-terreux en un composé de formule ($I_c$) :

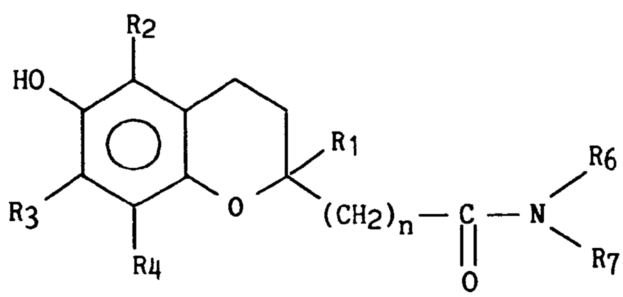

($I_c$)

avec $R_1$, $R_2$, $R_3$, $R_4$, $R_6$, $R_7$, et n tels que définis précédemment,

cas particulier des composés de formule (I) pour lesquels $R_6$ représente un atome d'hydrogène et X représente un atome d'oxygène, composé ($I_c$) qui peut être ensuite :

- ou bien réduit par action d'un hydrure mixte de metal alcalin en un dérivé de formule ($I_d$) :

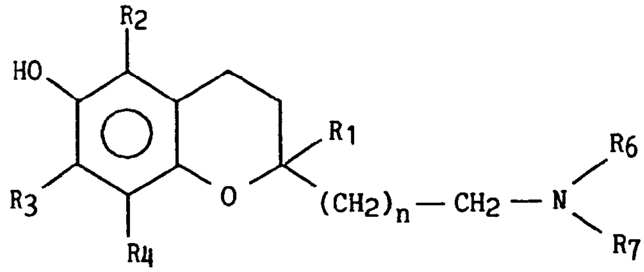

**(Id)**

avec $R_1$, $R_2$, $R_3$, $R_4$, $R_6$, $R_7$, et n tels que définis précédemment, cas particulier des composés de formule (I) pour lesquels $R_5$ représente un atome d'hydrogène et X représente 2 atomes d'hydrogène,

- ou bien éthérifié ou estérifié, afin d'obtenir, si on le désire, un groupement $R_5$ différent de celui présent dans la formule ( Ib), par action d'un dérivé de formule $R_{5'''}$-O- $R_{5'''}$ ou $R_{5'''}$-Hal, où Hal représente un atome d'halogène et $R_{5'''}$ représente un groupement acyle inférieur $R_a$-CO-, un groupement alcoxyalkyle de forme $R_a$-O-$R_b$-, un groupement alcoxycarbonylalkyle de forme $R_a$-O-CO-$R_b$-, ou un groupement carboxylalkyle de forme HOOC-$R_a$-, avec $R_a$ et $R_b$ tels que définis précédemment, pour obtenir un compose de formule ($I_e$) :

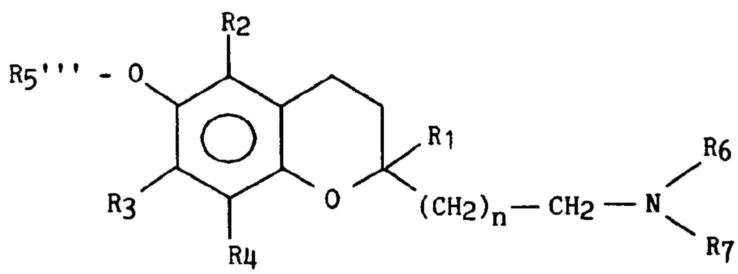

**(Ie)**

avec $R_1$, $R_2$, $R_3$, $R_4$, $R_5$, $R_7$, n, et $R_{5'''}$ tels que définis précédemment, cas particulier des composés de formule (I) pour lesquels $R_5$ représente un groupement $R_{5'''}$ et X représente 2 atomes d'hydrogène,

les composés de formule ($I_a$), ($I_b$), ($I_c$), ($I_d$), et ($I_e$) formant l'ensemble des composés de formule (I) qui peuvent être, si on le désire, purifiés et/ou séparés en leurs isomères optiques,

ces composés de formule (I) pouvant également être, le cas échéant, transformés en leurs sels d'addition à une base ou à un acide pharmaceutiquement acceptable.

**2)** Procédé de préparation selon la revendication 1 des composés de formule (I) pour lesquels $R_5$ représente un atome d'hydrogène, de leurs isomères optiques et de leurs sels d'addition à une base ou à un acide pharmaceutiquement acceptable.

**3)** Procédé de préparation selon la revendication 1 des composés de formule (I) pour lesquels X représente un atome d'oxygène, de leurs isomères optiques et de leurs éventuels sels d'addition à une base ou à un acide pharmaceutiquement acceptable.

**4)** Procédé de préparation selon la revendication 1 des composés de formule (I) pour lesquels $R_5$ représente un atome d'hydrogène et X représente un atome d'oxygène, de leurs isomères optiques et de leurs éventuels sels d'addition à une base ou à un acide pharmaceutiquement acceptable.

**5)** Procédé de préparation selon la revendication 1 des composés de formule (I) pour lesquels au moins 1 des 2 substituants $R_5$ ou $R_7$ représente un groupement pyridinyle ou un groupement pyridinyle substitué, de leurs isomères optiques et de leurs éventuels sels d'addition à une base ou à un acide pharmaceutiquement acceptable.

**6)** Procédé de préparation selon la revendication 1 des composés de formule (I) pour lesquels au moins 1 des 2 substituants $R_5$ ou $R_7$ représente un groupement quinoléinyle ou un groupement quinoléinyle substitué, de leurs isomères optiques et de leurs éventuels sels d'addition à une base ou à un acide pharmaceutiquement acceptable.

**7)** Procédé de préparation selon la revendication 1 des composés de formule (I) pour lesquels au moins 1 des 2 substituants $R_5$ ou $R_7$ représente un groupement phényle ou un groupement phényle substitué, de leurs isomères optiques et de leurs éventuels sels d'addition à une base ou à un acide pharmaceutiquement acceptable.

**8)** Procédé de préparation selon la revendication 1 du N-isobutyl N-(4,6-diméthylpyridin-2-yl) (6-hydroxy-3,4-dihydro-2,5,7,8-tétraméthyl-2H[1]-benzopyran-2-yl) carboxamide, de ses isomères optiques et de ses sels

d'addition à une base pharmaceutiquement acceptable.

**9)** Procédé de préparation selon la revendication 1 du N-(3,4,5-triméthoxyphényl) (6-hydroxy-3,4-dihydro-2,5,7,8-tétraméthyl-2H[1]-benzopyran-2-yl) carboxamide, de ses isomères optiques et de ses sels d'addition à une base pharmaceutiquement acceptable.

**10)** Procédé de préparation selon la revendication 1 du N-(4-méthylquinoléin-2-yl) (6-hydroxy-3,4-dihydro-2,5,7,8-tétraméthyl-2H[1]-benzopyran-2-yl) carboxamide, de ses isomères optiques et de ses sels d'addition à une base pharmaceutiquement acceptable.

**11)** Procédé de préparation selon la revendication 1 du N-{4-[(2,4-dioxo-5-thiazolidinyl)méthyl]phényl} (6-hydroxy-3,4-dihydro-2,5,7,8-tétraméthyl-2H [1]-benzopyran-2-yl) carboxamide, de ses isomères optiques et de ses sels d'addition à une base pharmaceutiquement acceptable.

**12)** Procédé de préparation selon la revendication 1 du N-(méthoxyéthyl) (6-hydroxy-3,4-dihydro-2,5,7,8-tétraméthyl-2H[1]-benzopyran-2-yl) carboxamide, de ses isomères optiques et de ses sels d'addition à une base pharmaceutiquement acceptable.

**13)** Procédé de préparation d'une composition pharmaceutique contenant comme principe actif au moins un composé préparé selon l'une des revendications 1 à 12, seul ou en combinaison avec un ou plusieurs excipients inertes non toxiques.

**14)** Procédé de préparation selon la revendication 13 d'une composition pharmaceutique utilisable dans le traitement et la prévention des affections dues ou reliées à des phènomènes de peroxydation ou de dérèglement de la biosynthèse des prostanoïdes, c'est à dire les désordres ischémiques centraux ou périphériques, les maladies inflammatoires, l'arthrite rhumatoïde, les maladies métaboliques, l'athérome, l'artériosclérose, les maladies respiratoires, l'asthme, l'emphysème, les maladies d'origine immunologique, le lupus érythémateux, les réactions allergiques, certains cancers, le vieillissement cérébral ou cutané, ainsi que dans la prévention et le traitement des dommages dus aux traumatismes chirurgicaux tels que la reperfusion d'organes.

**Revendications pour l'Etat contractant suivant : ES**

**1)** Procédé de préparation des composés de formule générale (I) :

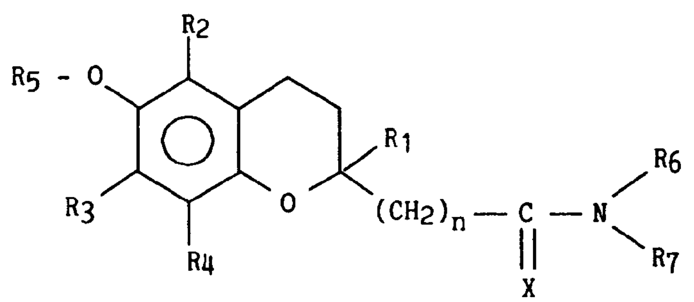

(I)

dans laquelle :
n représente un nombre entier égal à 0 ou 1,
X représente un atome d'oxygène ou 2 atomes d'hydrogène,

$R_1$, $R_2$, $R_3$, $R_4$, identiques ou différents, représentent chacun indépendamment l'un de l'autre un atome d'hydrogène ou un radical alkyl inférieur $R_a$-, où $R_a$ représente un groupement alkyle linéaire où ramifié de 1 à 8 atomes de carbone,

$R_5$ représente :
- un atome d'hydrogène,
- un groupement alkyle inférieur $R_a$-,
- un groupement acyle inférieur $R_a$-CO-,
- un groupement alcoxyalkyle de forme $R_a$-O-$R_b$-,
- un groupement alcoxycarbonyle de forme $R_a$-O-CO-,
- un groupement alcoxycarbonylalkyle de forme $R_a$-O-CO-$R_b$-,
- un groupement carboxylalkyle de forme HOOC-$R_a$-,

où $R_a$ et $R_b$, identiques ou différents, représentent chacun indépendamment l'un de l'autre, un radical alkyle linéaire ou ramifié comportant de 1 à 8 atomes de carbone,

$R_6$ et $R_7$

- soit :

forment ensemble avec l'atome d'azote qui les porte un groupement E ou un groupement E substitué

avec E représentant un groupement hétérocyclique spirannique de 8 à 12 chaînons incluant dans son squelette carboné de 1 à 4 hétéroatomes choisis parmi azote, soufre, et oxygène,

- soit :

identiques ou différents, représentent chacun indépendamment l'un de l'autre :

- un atome d'hydrogène,
- un groupement alkyle inférieur Ra- ou un groupement alkyle inférieur Ra- substitué,
- un groupement alcényle inférieur ou un groupement alcényle inférieur substitué, où le groupement alcényle représente un hydrocarbure insaturé, linéaire ou ramifié, comprenant 2 à 8 atomes de carbone,
- un groupement A-$(CH_2)_m$- ou un groupement A-$(CH_2)_m$- substitué, où m est un nombre entier égal à 0,1, ou 2 et A représente un groupement cycloalkyle comportant p atomes de carbone avec p étant un nombre entier de 3 à 7 étant entendu que si p est 3 ou 4 alors m peut être 0, 1, ou 2 et si p est 5,6, ou 7 alors m ne peut être que 1 ou 2,
- un groupement alcoxyalkyle de forme $R_a$-O-$R_b$- ou un groupement alcoxyalkyle de forme $R_a$-O-$R_b$- substitué, où $R_a$ et $R_b$, identiques ou différents, représentent un radical alkyle inférieur, linéaire ou ramifié de, 1 à 8 atomes de carbone,
- un groupement alcoxycarbonylalkyle de forme $R_a$-O-CO-$R_b$- ou un groupement alcoxycarbonylalkyle de forme $R_a$-O-CO-$R_b$- substitué, avec $R_a$ et $R_b$ tels que définis ci-dessus,
- un groupement B-$(CH_2)_q$- ou un groupement B-$(CH_2)_q$- substitué, où q est un nombre entier égal à 0, 1, 2, ou 3 et B représente un radical naphtalène, 1,3-dioxane , pyrane, ou benzopyrane,
- un groupement E-$(CH_2)_q$- ou un groupement E-$(CH_2)_q$- substitué, avec q et E tels que définis précédemment,
- un groupement phényl-$(CH_2)_q$- ou un groupement phényl-$(CH_2)_q$- substitué, avec q tel que défini ci-dessus,
- un groupement hétéroaryl-$(CH_2)_q$- ou un groupement hétéroaryl-$(CH_2)_q$- substitué, avec q tel que défini ci-dessus et où l'hétéroaryle est choisi parmi :

furane, quinoléine, isoquinoléine, pyridine, thiophène, thiazole, isothiazole, oxazole, isoxazole, naphtyridine, benzofurane, β-carboline, ou γ-carboline,

- un des radicaux $D_1$ à $D_9$ suivants ou un des radicaux $D_1$ à $D_9$ suivants substitué :

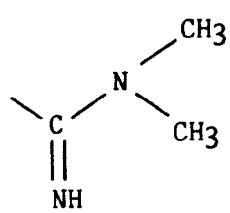 ,

$D_1$

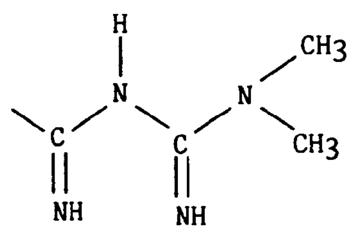 ,

$D_2$

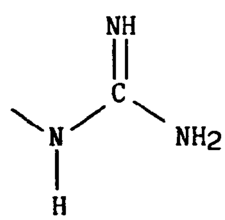 ,

$D_3$

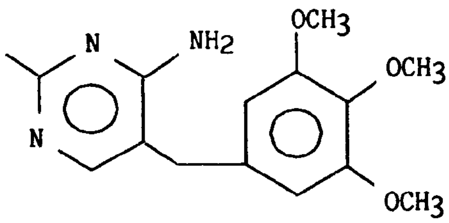

D4

,

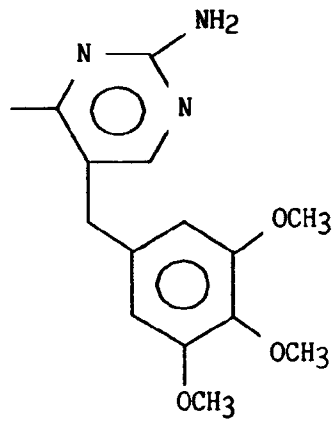

D5

,

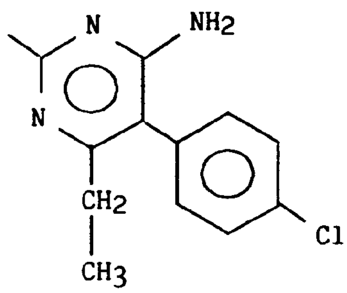

D6

,

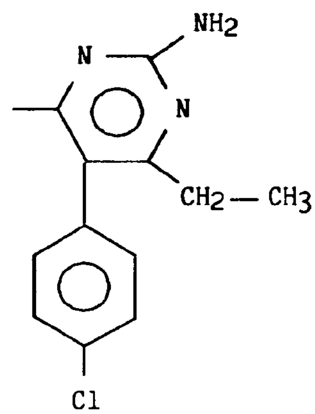

D7

,

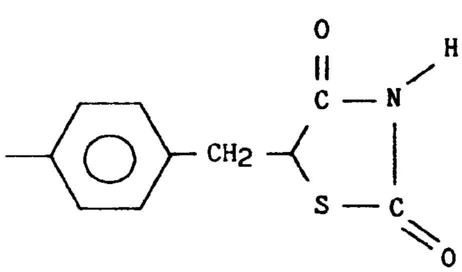

D8

,

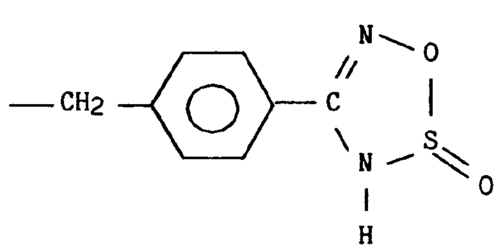

D9

,

étant entendu que

lorsque l'un des substituants $R_6$ ou $R_7$ représente un atome d'hydrogène, un groupement alkyle inférieur

comportant au maximum 3 atomes de carbone, un groupement phényl-$(CH_2)_{q'}$- non substitué, un groupement phényl-$(CH_2)_{q'}$- substitué par 1 ou 2 radicaux, un groupement pyridinyl-$(CH_2)_q$- non substitué, ou un groupement pyridinyl-$(CH_2)_q$- substitué par 1 ou 2 radicaux, avec q tel que défini précédemment et q' étant un nombre entier égal à 1, 2, ou 3,

alors l'une au moins des 2 conditions suivantes est vérifiée :

- ou bien l'autre substituant, $R_6$ ou $R_7$ selon le cas, ne représente pas : un atome d'hydrogène, un groupement alkyle inférieur comportant au maximum 3 atomes de carbone, un groupement phényl-$(CH_2)_{q'}$- non substitué, un groupement phényl-$(CH_2)_{q'}$- substitué par 1 ou 2 radicaux, un groupement pyridinyl-$(CH_2)_q$- non substitué, un groupement pyridinyl-$(CH_2)_q$- substitué par 1 ou 2 radicaux, avec q et q' tels que définis précédemment,
- ou bien le substituant $R_5$ ne représente pas un atome d'hydrogène, un groupement alkyle inférieur comportant au maximum 4 atomes de carbone, ou un groupement acyle inférieur $R'_5$-CO- avec $R'_5$ représentant un radical alkyle inférieur comportant au maximum 4 atomes de carbone,

étant entendu que

lors de cette description de la formule générale (I), le terme "substitué" affectant les groupements tels que définis précédemment : alkyle inférieur Ra-, alcényle inférieur, A-$(CH_2)_m$-, alcoxyalkyle $R_a$-O-$R_b$-, alcoxycarbonylalkyle $R_a$-O-CO-$R_b$-, B-$(CH_2)_q$-, E-$(CH_2)_q$-, phényl-$(CH_2)_q$-, phényl-$(CH_2)_{q'}$-, hétéroaryl-$(CH_2)_q$, $D_1$ à $D_9$, signifie, lorsque cela n'est pas précisé, que ces groupements peuvent être substitués par un ou plusieurs radicaux, identiques ou différents, et représentant chacun indépendamment l'un de l'autre :

un groupement alkyle inférieur $R_c$,
alcoxy inférieur $R_c$-O-,
acyle inférieur $R_c$-CO-,
trifluorométhyle,
carboxyle,
hydroxyle,
amino,
amino substitué par 1 ou 2 groupement alkyles inférieurs $R_c$-,
nitro,
oxo,
phényle,
alkylthio inférieur $R_c$-S- ,
thiol,
ou un atome d'halogène,

où $R_c$ représente un groupement alkyle linéaire ou ramifié de 1 à 6 atomes de carbone,

de leurs isomères optiques,

ainsi que, le cas échéant, de leurs sels d'addition à un acide ou à une base pharmaceutiquement acceptable,

caractérisé en ce que :

on utilise comme matière première un composé de formule (II) :

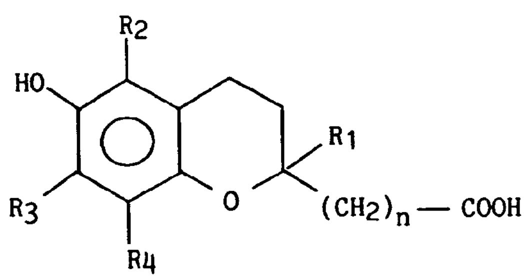

(II)

avec $R_1$, $R_2$, $R_3$, $R_4$, et n ayant la même définition que dans la formule générale (I),

que l'on peut éthérifier ou estérifier, en milieu basique anhydre, par un composé $R_{5''}$-Hal ou $R_{5''}$-O-$R_{6''}$, où Hal représente un atome d'halogène et où $R_{6''}$ représente un groupement alkyle inférieur $R_a$-, un groupement acyle inférieur $R_a$-CO-, un groupement alcoxyalkyle de forme $R_a$-O-$R_b$-, un groupement alcoxycarbonyle de forme $R_a$-O-CO-, un groupement alcoxycarbonylalkyle de forme $R_a$-O-CO-$R_b$-, un groupement carboxylalkyle de forme HOOC-$R_a$-, formules dans lesquelles $R_a$ et $R_b$, identiques ou différents, représentent chacun indépendamment l'un de l'autre un groupement alkyle inférieur, linéaire ou ramifié, comprenant de 1 à 8 atomes de carbone,

pour obtenir un composé de formule (III) :

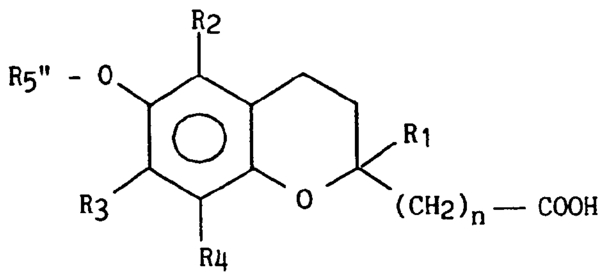

(III)

avec $R_1$, $R_2$, $R_3$, $R_4$, n, et $R_{5''}$ tels que définis précédemment, qui est transformé en son chlorure par action d'un agent d'halogénation, puis traité, dans un solvant approprié, en présence d'un agent alcalin, par une amine de formule (IV) :

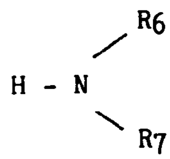

(IV)

avec $R_6$ et $R_7$ ayant la même signification que dans la formule (I) pour obtenir un composé de formule ($I_a$) :

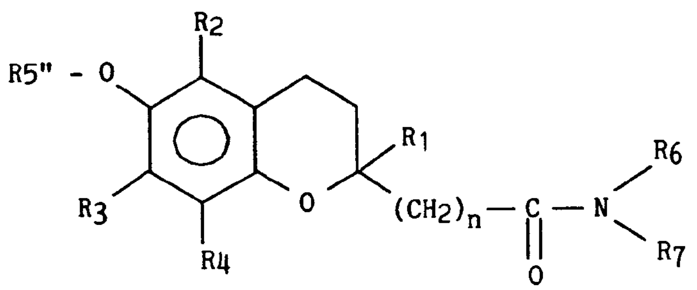

($I_a$)

avec $R_1$, $R_2$, $R_3$, $R_4$, $R_6$, $R_7$, n, et $R_{6''}$ tels que définis précédemment, cas particulier des composés de formule (I) pour lesquels $R_6$ représente un groupement $R_{6''}$ et X représente un atome d'oxygène,

que l'on peut ensuite :

- soit réduire par action d'un hydrure mixte de métal alcalin en un composé de formule ($I_b$) :

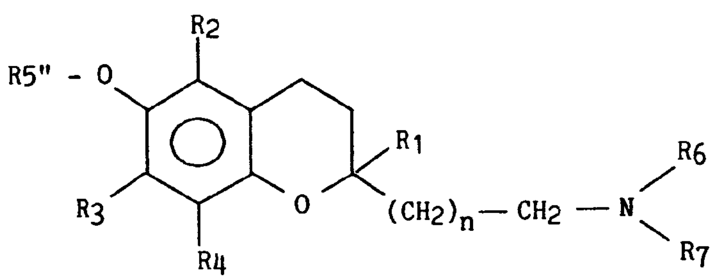

($I_b$)

avec $R_1$, $R_2$, $R_3$, $R_4$, $R_6$, $R_7$, n, et $R_{6''}$ tels que définis précédemment, cas particulier des composés de formule (I) pour lesquels $R_6$ représente un groupement $R_{6''}$ et X représente deux atomes d'hydrogène,
- soit saponifier par action d'un hydroxyde de métal alcalin ou alcalino-terreux en un composé de formule ($I_c$) :

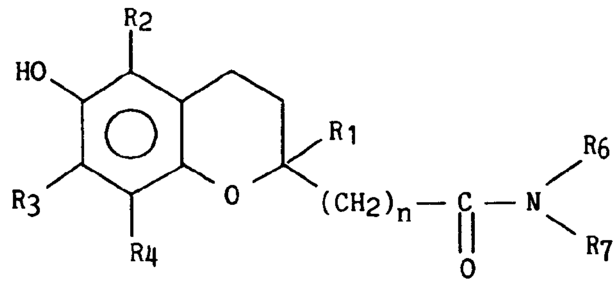

(Ic)

avec $R_1$, $R_2$, $R_3$, $R_4$, $R_6$, $R_7$, et n tels que définis précédemment,

cas particulier des composés de formule (I) pour lesquels $R_5$ représente un atome d'hydrogène et X représente un atome d'oxygène, composé ($I_c$) qui peut être ensuite :

- ou bien réduit par action d'un hydrure mixte de métal alcalin en un dérivé de formule ($I_d$) :

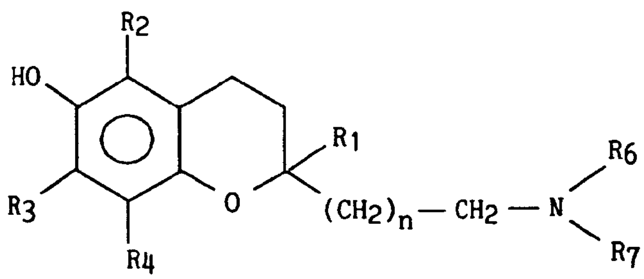

(Id)

avec $R_1$, $R_2$, $R_3$, $R_4$, $R_6$, $R_7$, et n tels que définis précédemment, cas particulier des composés de formule (I) pour lesquels $R_5$ représente un atome d'hydrogène et X représente 2 atomes d'hydrogène,

- ou bien éthérifié ou estérifié, afin d'obtenir, si on le désire, un groupement $R_5$ différent de celui présent dans la formule ( Ib), par action d'un dérivé de formule $R_{5'''}$-O- $R_{5'''}$ ou $R_{5'''}$-Hal, où Hal représente un atome d'halogène et $R_{5'''}$ représente un groupement acyle inférieur $R_a$-CO-, un groupement alcoxyalkyle de forme $R_a$-O-$R_b$-, un groupement alcoxycarbonylalkyle de forme $R_a$-O-CO-$R_b$-, ou un groupement carboxylalkyle de forme HOOC-$R_a$-, avec $R_a$ et $R_b$ tels que définis précédemment, pour obtenir un composé de formule ($I_e$) :

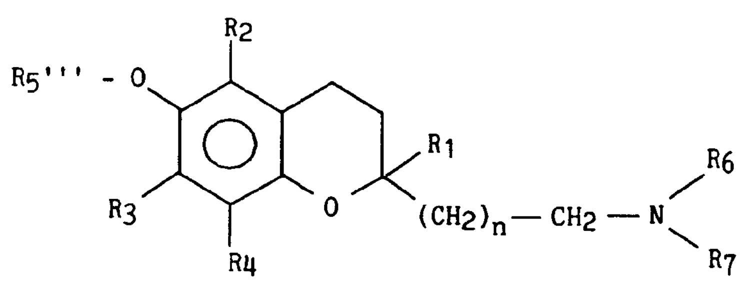

(Ie)

avec $R_1$, $R_2$, $R_3$, $R_4$, $R_5$, $R_7$, n, et $R_{5'''}$ tels que définis précédemment, cas particulier des composés de formule (I) pour lesquels $R_5$ représente un groupement $R_{5'''}$ et X représente 2 atomes d'hydrogène,

les composés de formule ($I_a$), ($I_b$), ($I_c$), ($I_d$), et ($I_e$) formant l'ensemble des composés de formule (I) qui peuvent être, si on le désire, purifiés et/ou séparés en leurs isomères optiques,

ces composés de formule (I) pouvant également être, le cas échéant, transformés en leurs sels d'addition à une base ou à un acide pharmaceutiquement acceptable.

**2)** Procédé de préparation selon la revendication 1 des composés de formule (I) pour lesquels $R_5$ représente un atome d'hydrogène, de leurs isomères optiques et de leurs sels d'addition à une base ou à un acide pharmaceutiquement acceptable.

**3)** Procédé de préparation selon la revendication 1 des composés de formule (I) pour lesquels X représente un atome d'oxygène, de leurs isomères optiques et de leurs éventuels sels d'addition à une base ou à un acide pharmaceutiquement acceptable.

**4)** Procédé de préparation selon la revendication 1 des composés de formule (I) pour lesquels $R_5$ repré-

sente un atome d'hydrogène et X représente un atome d'oxygène, de leurs isomères optiques et de leurs éventuels sels d'addition à une base ou à un acide pharmaceutiquement acceptable.

**5)** Procédé de préparation selon la revendication 1 des composés de formule (I) pour lesquels au moins 1 des 2 substituants $R_6$ ou $R_7$ représente un groupement pyridinyle ou un groupement pyridinyle substitué, de leurs isomères optiques et de leurs éventuels sels d'addition à une base ou à un acide pharmaceutiquement acceptable.

**6)** Procédé de préparation selon la revendication 1 des composés de formule (I) pour lesquels au moins 1 des 2 substituants $R_6$ ou $R_7$ représente un groupement quinoléinyle ou un groupement quinoléinyle substitué, de leurs isomères optiques et de leurs éventuels sels d'addition à une base ou à un acide pharmaceutiquement acceptable.

**7)** Procédé de préparation selon la revendication 1 des composés de formule (I) pour lesquels au moins 1 des 2 substituants $R_6$ ou $R_7$ représente un groupement phényle ou un groupement phényle substitué, de leurs isomères optiques et de leurs éventuels sels d'addition à une base ou à un acide pharmaceutiquement acceptable.

**8)** Procédé de préparation selon la revendication 1 du N-isobutyl N-(4,6-diméthylpyridin-2-yl) (6-hydroxy-3,4-dihydro-2,5,7,8-tétraméthyl-2H[1]-benzopyran-2-yl) carboxamide, de ses isomères optiques et de ses sels d'addition à une base pharmaceutiquement acceptable.

**9)** Procédé de préparation selon la revendication 1 du N-(3,4,5-triméthoxyphényl) (6-hydroxy-3,4-dihydro-2,5,7,8-tétraméthyl-2H[1]benzopyran-2-yl) carboxamide, de ses isomères optiques et de ses sels d'addition à une base pharmaceutiquement acceptable.

**10)** Procédé de préparation selon la revendication 1 du N-(4-méthylquinoléin-2-yl) (6-hydroxy-3,4-dihydro-2,5,7,8-tétraméthyl-2H[1]benzopyran-2-yl) carboxamide, de ses isomères optiques et de ses sels d'addition à une base pharmaceutiquement acceptable.

**11)** Procédé de préparation selon la revendication 1 du N-{4-[(2,4-dioxo-5-thiazolidinyl)methyl]phényl} (6-hydroxy-3,4-dihydro-2,5,7,8-tétraméthyl-2H[1]-benzopyran-2-yl) carboxamide, de ses isomères optiques et de ses sels d'addition à une base pharmaceutiquement acceptable.

**12)** Procédé de préparation selon la revendication 1 du N-(méthoxyéthyl) (6-hydroxy-3,4-dihydro-2,5,7,8-tétraméthyl-2H[1]-benzopyran-2-yl) carboxamide, de ses isomères optiques et de ses sels d'addition à une base pharmaceutiquement acceptable.

**13)** Procédé de préparation d'une composition pharmaceutique contenant comme principe actif au moins un composé préparé selon l'une des revendications 1 à 12, seul ou en combinaison avec un ou plusieurs excipients inertes non toxiques.

**14)** Procédé de préparation selon la revendication 13 d'une composition pharmaceutique utilisable dans le traitement et la prévention des affections dues ou reliées à des phénomènes de peroxydation ou de dérèglement de la biosynthèse des prostanoïdes, c'est à dire les désordres ischémiques centraux ou périphériques, les maladies inflammatoires, l'arthrite rhumatoïde, les maladies métaboliques, l'athérome, l'artériosclérose, les maladies respiratoires, l'asthme, l'emphysème, les maladies d'origine immunologique, le lupus érythémateux, les réactions allergiques, certains cancers, le vieillissement cérébral ou cutané, ainsi que dans la prévention et le traitement des dommages dus aux traumatismes chirurgicaux tels que la reperfusion d'organes.

Office européen des brevets

# RAPPORT DE RECHERCHE EUROPEENNE

Numero de la demande

EP 92 40 1225

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | EP-A-0 391 625 (PFIZER INC.)<br>* page 2 - page 5, ligne 10 *<br>--- | 1,14 | C 07 D 311/72<br>C 07 D 405/12<br>C 07 D 407/12<br>C 07 D 409/12<br>C 07 D 413/12<br>C 07 D 417/12<br>C 07 D 471/04<br>C 07 D 419/12<br>A 61 K 31/355 |
| A | EP-A-0 387 771 (ONO PHARMACEUTICAL CO. LTD)<br>* page 6, ligne 55 - page 9, ligne 45; page 34, ligne 40 - page 38 *<br>--- | 1,14 | |
| A | EP-A-0 369 874 (MERREL DOW PHARMACEUTICALS INC.)<br>* page 2, ligne 52; page 10 - page 15, ligne 31 *<br>--- | 1,14 | |
| A | EP-A-0 354 508 (F. HOFFMANN-LAROCHE AG)<br>* page 3 - page 5, ligne 22; page 8, ligne 55 - page 13, ligne 31 *<br>--- | 1,14 | |
| A | EP-A-0 293 078 (THE UPJOHN COMPANY)<br>* page 4, ligne 7 - page 13, ligne 5; page 55 - page 60, ligne 12 *; & WO - A - 8808424 (cat. D)<br>--- | 1,14 | **DOMAINES TECHNIQUES RECHERCHES (Int. Cl.5)** |
| A | US-A-4 178 380 (J.B. CARR)<br>* colonne 1 - colonne 2, ligne 5 *<br>--- | 1,14 | C 07 D |
| A | EP-A-0 277 836 (SANKYO COMPANY LIMITED)<br>* pages 2-11 *<br>----- | 1,14 | |

Le présent rapport a été établi pour toutes les revendications

| Lieu de la recherche | Date d'achèvement de la recherche | Examinateur |
|---|---|---|
| BERLIN | 11-08-1992 | KYRIAKAKOU G |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)